# EUROPEAN PATENT APPLICATION

(11) **EP 4 648 165 A1**
(43) Date of publication of application: **12.11.2025**
(21) Application number: 24753268.2
(22) Date of filing: 02.02.2024
(51) Int. Cl.: H01M 10/0567, C07D 251/32, H01M 10/052, H01M 10/054, H01M 10/0569

(54) **NONAQUEOUS ELECTROLYTE SOLUTION, NONAQUEOUS ELECTROLYTE BATTERY, AND COMPOUND**

(30) Priority: 06.02.2023 JP 2023016465
(71) Applicant: Central Glass Company, Limited, Ube-shi, Yamaguchi 755-0001 (JP)
(72) Inventor: IWASAKI, Susumu, Tokyo 101-0054 (JP); TERADA, Ryosuke, Tokyo 101-0054 (JP); KATAOKA, Fuga, Tokyo 101-0054 (JP); KAWABATA, Wataru, Tokyo 101-0054 (JP); TAKAHASHI, Mikihiro, Tokyo 101-0054 (JP); MORINAKA, Takayoshi, Tokyo 101-0054 (JP)
(74) Representative: Manitz Finsterwald Patent- und Rechtsanwaltspartnerschaft mbB
(86) International application number: PCT/JP2024/003558
(87) International publication number: WO 2024/166825

(57) **Abstract**

The present disclosure provides a nonaqueous electrolyte solution containing at least one selected from the group consisting of a compound represented by the following general formula (1) and a compound represented by the following general formula (2): 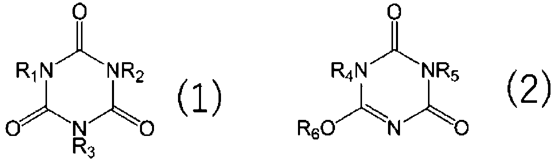 and a nonaqueous electrolyte solution battery including at least a positive electrode, a negative electrode, a separator, and the nonaqueous electrolyte solution. In the general formula (1), R₁ to R₃ represent groups which may be the same or different and at least one of R₁ to R₃ represents a -SO₂F group. In the general formula (2), R₄ to R₆ represent groups which may be the same or different and at least one of R₄ to R₆ represents a -SO₂F group.

## Description

### TECHNICAL FIELD

The present disclosure relates to a nonaqueous electrolyte solution, a nonaqueous electrolyte solution battery, and compounds.

### BACKGROUND ART

As measures for improving the durability such as cycle characteristics and high-temperature storage characteristics of a nonaqueous electrolyte solution battery, optimization of various constituting elements of the battery including active materials of a positive electrode and a negative electrode has been hitherto studied. Techniques relating to a nonaqueous electrolyte solution have also been studied, and use of a variety of additives to prevent deterioration due to decomposition of the nonaqueous electrolyte solution at surfaces of active positive and negative electrodes has been proposed.

Patent Literature 1 discloses a nonaqueous electrolyte solution for a lithium battery as a nonaqueous electrolyte solution having excellent electrochemical characteristics, for example, a long cycle life, a high capacity retention rate, and excellent storage stability. To the nonaqueous electrolyte solution, a compound in which alkyl groups substituted with a fluorosulfonyl group are bonded to the three nitrogen atoms of an isocyanurate ring is added.

Further, Patent Literature 2 discloses a nonaqueous electrolyte solution to which a compound in which vinylsulfonyl groups are bonded to the three nitrogen atoms of an isocyanurate ring is added, as a nonaqueous electrolyte solution capable of improving the cycle characteristics of a secondary battery.

### CITATION LIST

### PATENT LITERATURE

Patent Literature 1: JP7076527B
Patent Literature 2: JP2019-50135A

### SUMMARY OF INVENTION

### TECHNICAL PROBLEM

However, as a result of investigation by the present inventors, it has been found that, when these isocyanuric acid derivatives are contained, an increase in an internal resistance of the battery is observed, and there is no or small effect of improving an initial input/output characteristic. Thus, there is room for further investigation of resistance characteristics, particularly of initial resistance characteristics.

The present disclosure has been made in view of the above circumstances, and an object thereof is to provide a nonaqueous electrolyte solution which can reduce the initial resistance value of a battery and a nonaqueous electrolyte solution battery. Another object is to provide a compound which can be suitably used in the above nonaqueous electrolyte solution.

### SOLUTION TO PROBLEM

In view of such a problem, the present inventors have intensively studied and found that the initial resistance value of a nonaqueous electrolyte solution battery can be reduced using a nonaqueous electrolyte solution containing at least one selected from the group consisting of a compound represented by the general formula (1) and a compound represented by the general formula (2), which will be described later, and the inventors have thus solved the above problem.

That is, the present inventors have found that the above problem can be solved by the following configurations.
[1]
   A nonaqueous electrolyte solution containing at least one selected from the group consisting of a compound represented by the following general formula (1) and a compound represented by the following general formula (2). 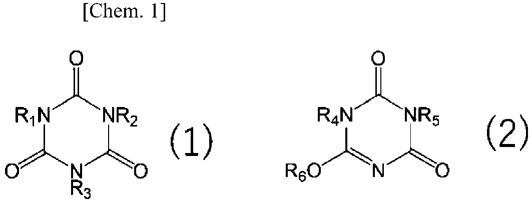 [In the general formula (1), R₁ to R₃ represent groups which may be the same or different and at least one of R₁ to R₃ represents a -SO₂F group.
   In the general formula (2), R₄ to R₆ represent groups which may be the same or different and at least one of R₄ to R₆ represents a -SO₂F group.]
[2]
   The nonaqueous electrolyte solution according to [1], in which one or two of R₁ to R₃ in the general formula (1) represent a -SO₂F group.
[3]
   The nonaqueous electrolyte solution according to [1] or [2], in which one or two of R₁ to R₃ in the general formula (1) represent a -SO₂F group, and when a plurality of R₁ to R₃ are other than a -SO₂F group, the plurality of R₁ to R₃ each independently represent a group selected from the group consisting of a hydrogen atom, an alkali metal cation, an alkyl group, an alkenyl group, and an alkynyl group.
[4]
   The nonaqueous electrolyte solution according to any one of [1] to [3], in which R₁ to R₃ in the general formula (1) all represent a -SO₂F group.
[5]
   The nonaqueous electrolyte solution according to any one of [1] to [4], in which one or two of R₄ to R₆ in the general formula (2) represent a -SO₂F group.
[6]
   The nonaqueous electrolyte solution according to any one of [1] to [5] in which R₆ in the general formula (2) represents a -SO₂F group.
[7]
   A nonaqueous electrolyte solution battery at least including a positive electrode, a negative electrode, a separator, and the nonaqueous electrolyte solution according to any one of [1] to [6].
[8]
   A compound represented by the following general formula (11). 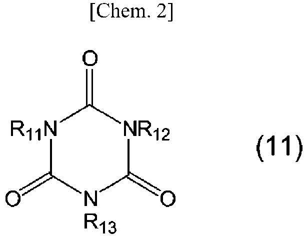 [In the general formula (11), one or two of R₁₁ to R₁₃ represent a -SO₂F group, and when a plurality of R₁₁ to R₁₃ are other than a -SO₂F group, the plurality of R₁₁ to R₁₃ each independently represent a group selected from the group consisting of a hydrogen atom, an alkali metal cation, an alkyl group, an alkenyl group, and an alkynyl group.]
[9]
   A compound represented by the following general formula (2). 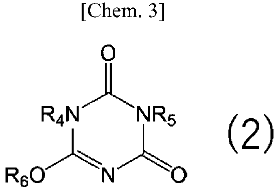 [In the general formula (2), R₄ to R₆ represent groups which may be the same or different and at least one of R₄ to R₆ represents a -SO₂F group.]

### ADVANTAGEOUS EFFECTS OF INVENTION

According to the present disclosure, a nonaqueous electrolyte solution which can reduce the initial resistance value of a battery and a nonaqueous electrolyte solution battery can be provided. Moreover, compounds which can be suitably used in the nonaqueous electrolyte solution can be provided.

### DESCRIPTION OF EMBODIMENTS

Configurations and combinations thereof in the following embodiments are merely examples, and additions, replacements, and other changes of the configurations may be made without departing from the scope of the present disclosure. In addition, the present disclosure is not limited by the embodiments but is only limited by the scope of claims.

The term "to" in the present specification is used with the meanings including the numerical values indicated before and after "to" as a lower limit value and an upper limit value.

In the present specification, an initial resistance value means a resistance value of a nonaqueous electrolyte solution battery immediately after an initial charge and discharge operation for battery stabilization. Specifically, the initial resistance value refers to a resistance value obtained by initial impedance measurement after three cycles of a charge and discharge operation for battery stabilization.

### [1. Nonaqueous Electrolyte Solution]

The nonaqueous electrolyte solution of the present disclosure is a nonaqueous electrolyte solution containing at least one selected from the group consisting of the compound represented by the general formula (1) and the compound represented by the general formula (2) (hereinafter sometimes referred to as "component (I)").

When the nonaqueous electrolyte solution containing the above component (I) is used in a nonaqueous electrolyte solution battery (for example, a lithium ion secondary battery or a sodium ion secondary battery), the component (I) decomposes on at least one of the positive electrode and the negative electrode and forms a film having excellent cation conductivity on the surface of at least one of the positive electrode and the negative electrode. It is considered that this film prevents direct contact between the nonaqueous organic solvent or the solute which can be contained in the nonaqueous electrolyte solution and the electrode active material and reduces the cation dissociation energy of the solute. The present inventors presume that, as a result, an effect of reducing the initial resistance of the nonaqueous electrolyte solution battery is exhibited.

### <Component (I)>

The compound represented by the general formula (1) is described below.

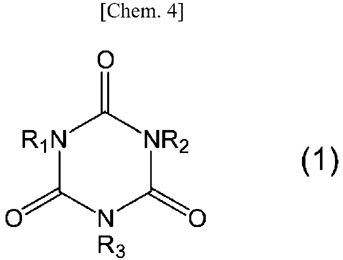

In the general formula (1), R₁ to R₃ represent groups which may be the same or different. Here, at least one of R₁ to R₃ represents a -SO₂F group.

Examples of the groups represented by R₁ to R₃ include a hydrogen atom, an alkali metal cation, a halogen atom, an alkyl group, an alkenyl group, an alkynyl group, a cycloalkyl group, a cycloalkenyl group, an aryl group, - S(=O)₂-R^{a}, -P(=O)-R^{b}R^{c}, -Si-R^{d}R^{c}R^{f}, and -C(=O)-R^{g}. R^{a} to R^{g} each independently represent a halogen atom, or an organic group selected from the group consisting of an alkyl group, an alkenyl group, an alkynyl group, a cycloalkyl group, a cycloalkenyl group, an aryl group, an alkoxy group, an alkenyloxy group, an alkynyloxy group, a cycloalkoxy group, a cycloalkenyloxy group, an aryloxy group, a monoalkylamino group, a dialkylamino group, and an alkoxycarbonyl group, and the organic group can contain at least one of an oxygen atom and an unsaturated bond. Moreover, R^{b} and R^{c} can bond to each other to form a cyclic structure.

When R₁, R₂, or R₃ represents an alkali metal cation, the bond between the nitrogen atom in the general formula (1) and R₁, R₂, or R₃ represents an ionic bond.

In the groups represented by R₁ to R₃, at least any one hydrogen atom may be substituted with a substituent such as a halogen atom, a cyano group, and an acryloyloxy group. Examples of the halogen atom include a fluorine atom, a bromine atom, an iodine atom, and the like, and the halogen atom may be a fluorine atom.

Examples of the alkali metal cation represented by R₁ to R₃ include a lithium ion, a sodium ion, a potassium ion, and the like. Of these, the alkali metal cation may be a lithium ion or a sodium ion.

Examples of the halogen atom represented by R₁ to R₃ include a fluorine atom, a chlorine atom, a bromine atom, and the like. Of these, the halogen atom may be a fluorine atom or a chlorine atom and, in particular, a fluorine atom.

The alkyl group represented by R₁ to R₃ may be an alkyl group having 1 to 10 carbon atoms, and examples thereof include a methyl group, an ethyl group, an n-propyl group, an isopropyl group, an n-butyl group, a sec-butyl group, an isobutyl group, a tert-butyl group, an n-pentyl group, an n-hexyl group, an n-octyl group, an n-decanyl group, and the like. Of these, the alkyl group may be an alkyl group having 1 to 3 carbon atoms or may be a methyl group or an ethyl group.

The alkenyl group represented by R₁ to R₃ may be an alkenyl group having 2 to 10 carbon atoms, and examples thereof include a vinyl group, an allyl group, a I-propenyl group, an isopropenyl group, a 2-butenyl group, a 1,3-butadienyl group, and the like. Of these, the alkenyl group may be an alkenyl group having 2 or 3 carbon atoms.

The alkynyl group represented by R₁ to R₃ may be an alkynyl group having 2 to 10 carbon atoms, and examples thereof include an ethynyl group, a 2-propynyl group, a 1,1-dimethyl-2-propynyl group, and the like. Of these, the alkynyl group may be an alkynyl group having 2 or 3 carbon atoms.

The cycloalkyl group represented by R₁ to R₃ may be a cycloalkyl group having 3 to 10 carbon atoms, and examples thereof include a cyclopentyl group, a cyclohexyl group, and the like. Of these, the cycloalkyl group may be a cyclohexyl group.

The cycloalkenyl group represented by R₁ to R₃ may be a cycloalkenyl group having 3 to 10 carbon atoms, and examples thereof include a cyclopentenyl group, a cyclohexenyl group, and the like.

The aryl group represented by R₁ to R₃ may be an aryl group having 6 to 10 carbon atoms, and examples thereof include a phenyl group, a tolyl group, a xylyl group, and the like. Of these, the aryl group may be a phenyl group.

Examples of the halogen atom represented by R^{a} to R^{g} in the groups -S(=O)₂-R^{a}, -P(=O)-R^{b}R^{c}, -Si-R^{d}R^{e}R^{f}, and -C(=O)-R^{g} represented by R₁ to R₃ include a fluorine atom, a chlorine atom, a bromine atom, and the like.

Examples of the alkyl group, the alkenyl group, the alkynyl group, the cycloalkyl group, the cycloalkenyl group, and the aryl group represented by R^{a} to R^{g} include an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, and an aryl group having 6 to 10 carbon atoms, respectively, and specific examples thereof include the examples listed in the alkyl group having 1 to 10 carbon atoms, the alkenyl group having 2 to 10 carbon atoms, the alkynyl group having 2 to 10 carbon atoms, the cycloalkyl group having 3 to 10 carbon atoms, the cycloalkenyl group having 3 to 10 carbon atoms, and the aryl group having 6 to 10 carbon atoms as R₁ to R₃ above.

Examples of the alkoxy group represented by R^{a} to R^{g} include an alkoxy group having 1 to 10 carbon atoms, and examples of the alkyl group contained in the alkoxy group include the alkyl groups having 1 to 10 carbon atoms represented by R₁ to R₃.

Examples of the alkenyloxy group represented by R^{a} to R^{g} include an alkenyloxy group having 2 to 10 carbon atoms, and examples of the alkenyl group contained in the alkenyloxy group include the alkenyl groups having 2 to 10 carbon atoms represented by R₁ to R₃.

Examples of the alkynyloxy group represented by R^{a} to R^{g} include an alkynyloxy group having 2 to 10 carbon atoms, and examples of the alkynyl group contained in the alkynyloxy group include the alkynyl groups having 2 to 10 carbon atoms represented by R₁ to R₃.

Examples of the cycloalkoxy group represented by R^{a} to R^{g} include a cycloalkoxy group having 3 to 10 carbon atoms, and examples of the cycloalkyl group contained in the cycloalkoxy group include the cycloalkyl groups having 3 to 10 carbon atoms represented by R₁ to R₃.

Examples of the cycloalkenyloxy group represented by R^{a} to R^{g} include a cycloalkenyloxy group having 3 to 10 carbon atoms, and examples of the cycloalkenyl group contained in the cycloalkenyloxy group include the cycloalkenyl groups having 3 to 10 carbon atoms represented by R₁ to R₃.

Examples of the aryloxy group represented by R^{a} to R^{g} include an aryloxy group having 6 to 10 carbon atoms, and examples of the aryl group contained in the aryloxy group include the aryl groups having 6 to 10 carbon atoms represented by R₁ to R₃.

Examples of the monoalkylamino group represented by R^{a} to R^{g} include a monoalkylamino group containing an alkyl group having 1 to 10 carbon atoms, and examples of the alkyl group having 1 to 10 carbon atoms include the alkyl groups having 1 to 10 carbon atoms represented by R₁ to R₃.

Examples of the dialkylamino group represented by R^{a} to R^{g} include a dialkylamino group containing two alkyl groups having 1 to 10 carbon atoms, and examples of the alkyl groups having 1 to 10 carbon atoms include the alkyl groups having 1 to 10 carbon atoms represented by R₁ to R₃.

Examples of the alkoxycarbonyl group represented by R^{a} to R^{g} include an alkoxycarbonyl group having 2 to 11 carbon atoms, and examples of the alkyl group contained in the alkoxycarbonyl group include the alkyl groups having 1 to 10 carbon atoms represented by R₁ to R₃.

The organic group represented by R^{a} to R^{g} can contain an oxygen atom. For example, an oxygen atom may be included in a carbon atom-carbon atom bond in the organic group.

Specific examples of the organic group containing an oxygen atom in a carbon atom-carbon atom bond include a -CH₂CH₂-O-CH₃ group, a -CH₂CH₂-O-CH₂CH₃ group, and the like, for example.

R^{b} and R^{c} can bond to each other to form a cyclic structure. The cyclic structure formed by R^{b} and R^{c} together with the phosphorus atom may be a 5- or 6-membered ring or may be a 5-membered ring.

When R₁ to R₃ represent -S(=O)₂-R^{a}, R^{a} may be a fluorine atom, an alkyl group having 1 to 3 carbon atoms, an alkenyl group having 2 or 3 carbon atoms, or an alkoxy group having 1 to 3 carbon atoms, may be a fluorine atom, a methyl group, an ethyl group, or a methoxy group and may be a fluorine atom.

When R₁ to R₃ represent -P(=O)-R^{b}R^{c}, R^{b} and R^{c} may be each independently a fluorine atom, an alkyl group having 1 to 3 carbon atoms, or an alkoxy group having 1 to 3 carbon atoms, may be a fluorine atom or an alkoxy group having 1 to 3 carbon atoms and may be a fluorine atom or a methoxy group.

R^{b} and R^{c} may represent an alkoxy group having 1 or 2 carbon atoms and may bond to each other to form a 5-membered ring or a 6-membered ring together with the phosphorus atom.

When R₁ to R₃ represent -Si-R^{d}R^{e}R^{f}, R^{d}, R^{e}, and R^{f} may be each independently an alkyl group having 1 to 3 carbon atoms and may be a methyl group.

When R₁ to R₃ represent -C(=O)-R^{g}, R^{g} may be a fluorine atom, an alkyl group having 1 to 3 carbon atoms, an alkoxy group having 1 to 3 carbon atoms, a monoalkylamino group (the number of carbon atoms of the alkyl group contained in the group is 1 to 3), a dialkylamino group (the numbers of carbon atoms of the alkyl groups contained in the group are 1 to 3), or an alkoxycarbonyl group (the number of carbon atoms of the alkyl group contained in the group is 1 to 3) and may be a fluorine atom, a methyl group, an ethyl group, a methoxy group, an ethoxy group, a methylamino group, a dimethylamino group, or a methoxycarbonyl group.

At least one of R₁ to R₃ represents a -SO₂F group, but one or two thereof may be a -SO₂F group, and two thereof may be a -SO₂F group.

The group other than the -SO₂F group represented by R₁ to R₃ may be a group other than -S(=O)₂-R^{a} described above, and when there are a plurality of such groups, the groups may be each independently a group selected from the group consisting of a hydrogen atom, an alkali metal cation, an alkyl group, an alkenyl group, and an alkynyl group.

One or two of R₁ to R₃ represent a -SO₂F group, and when a plurality of groups thereof are other than a -SO₂F group, the groups may each independently represent a group selected from the group consisting of a hydrogen atom, an alkali metal cation, an alkyl group, an alkenyl group, and an alkynyl group.

That is, the compound represented by the general formula (1) may be a compound represented by the following general formula (11).

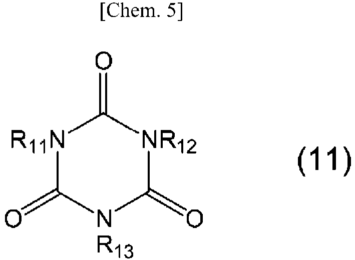

In the general formula (11), one or two of R₁₁ to R₁₃ represent a -SO₂F group, and when a plurality of groups thereof are other than a -SO₂F group, the groups each independently represent a group selected from the group consisting of a hydrogen atom, an alkali metal cation, an alkyl group, an alkenyl group, and an alkynyl group.

The alkali metal cation, the alkyl group, the alkenyl group, and the alkynyl group represented by R₁₁ to R₁₃ in the general formula (11) are synonymous with the alkali metal cation, the alkyl group, the alkenyl group, and the alkynyl group represented by R₁ to R₃ in the general formula (1), and specific examples thereof are also the same.

In particular, of R₁ to R₃ (R₁₁ to R₁₃ in the compound represented by the general formula (11)), two may represent a -SO₂F group, and one may be a group selected from the group consisting of a hydrogen atom, an alkali metal cation, an alkyl group, an alkenyl group, and an alkynyl group. Moreover, two thereof may represent a -SO₂F group, and one thereof may be an alkali metal cation.

Moreover, R₁ to R₃ may be all a -SO₂F group.

Specific examples of the compound represented by the general formula (1) are shown below, but the present invention is not limited thereto. In the following structural formulae, Me represents a methyl group, and Et represents an ethyl group.

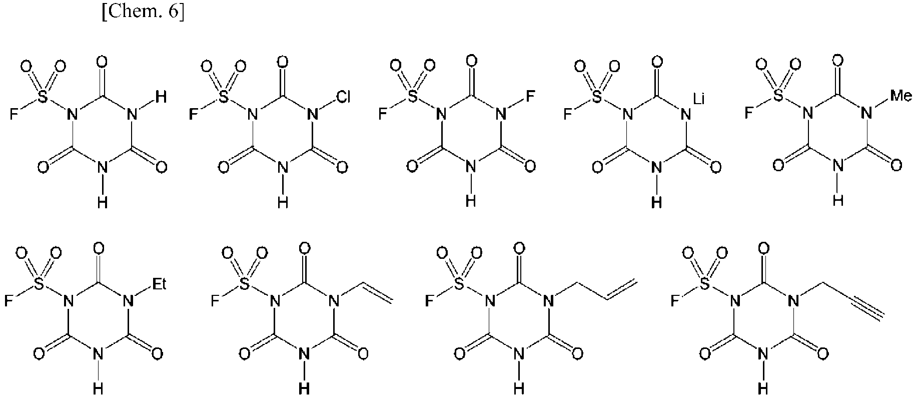

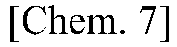

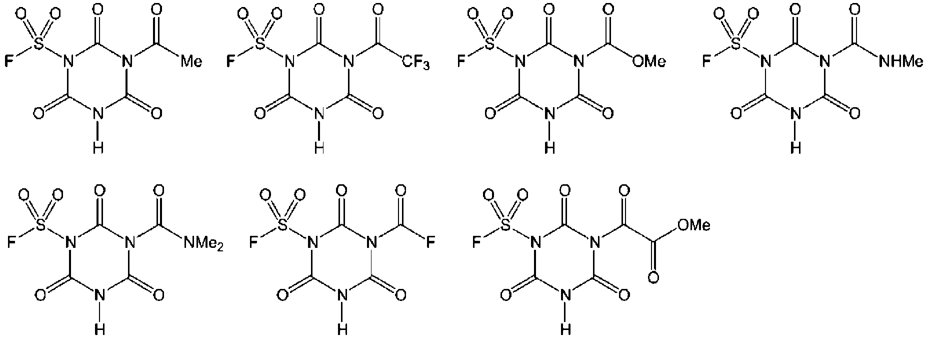

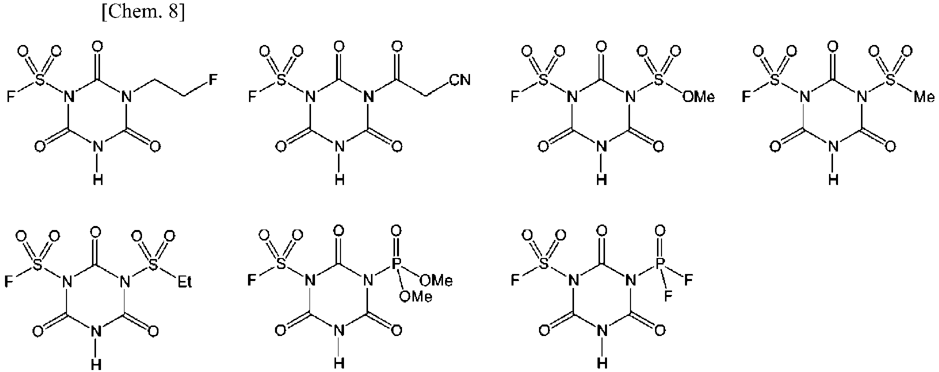

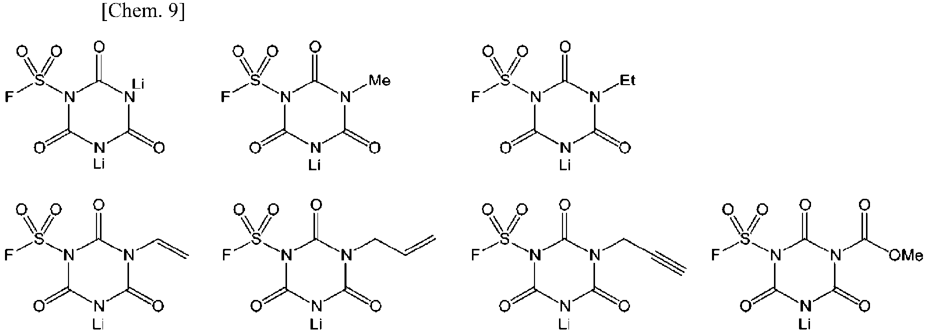

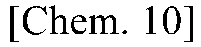

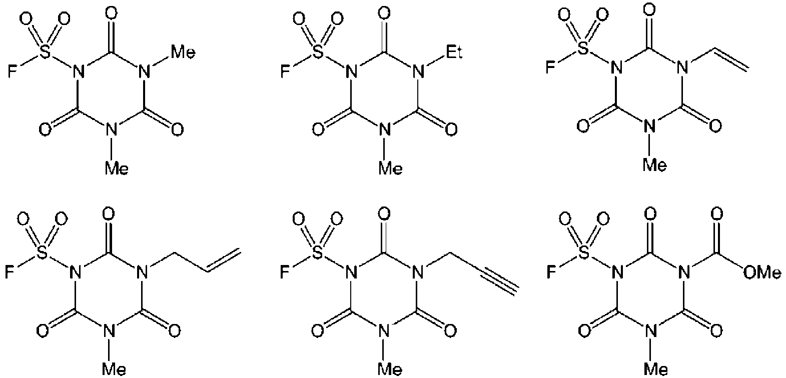

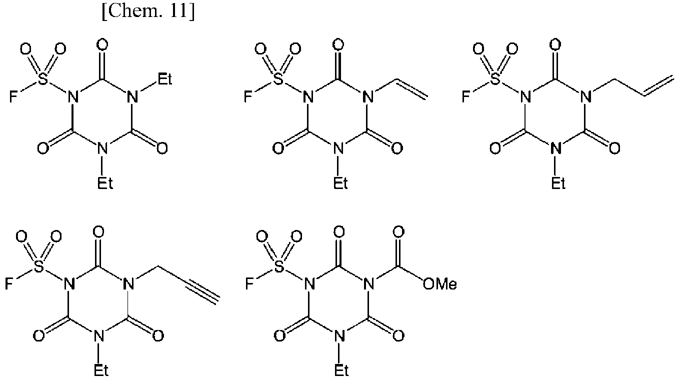

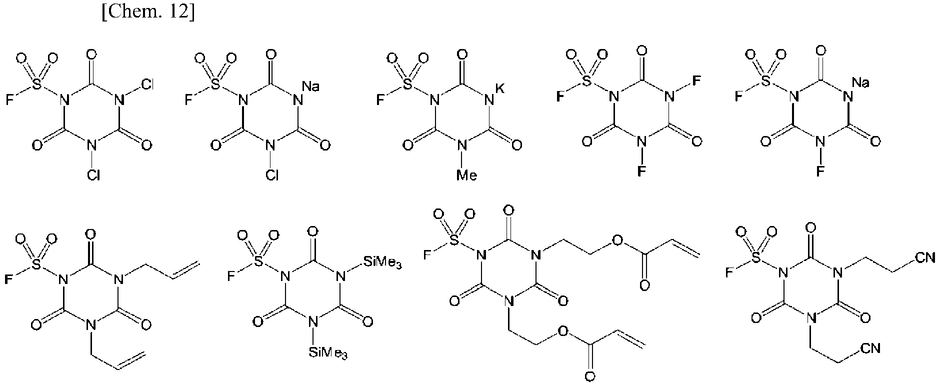

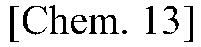

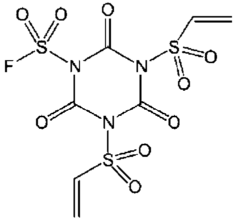

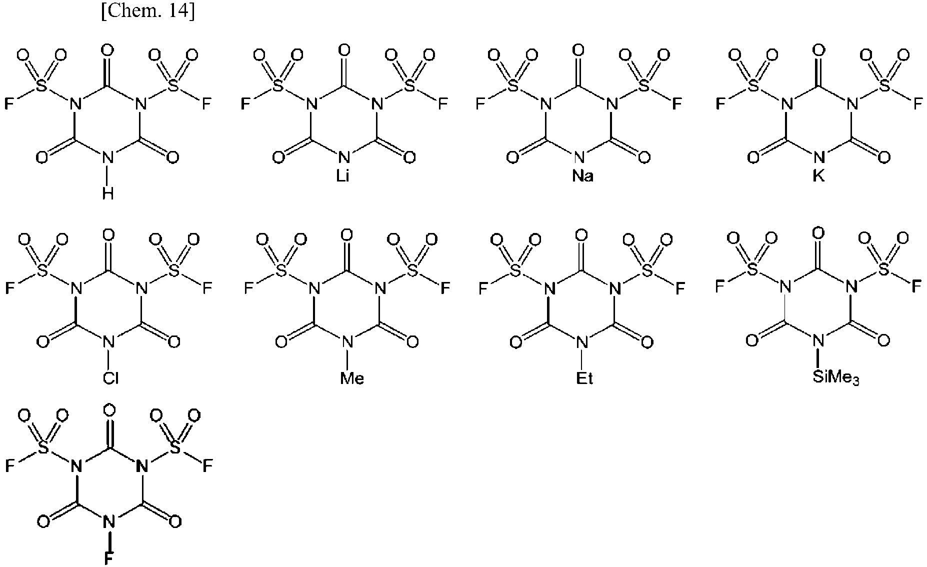

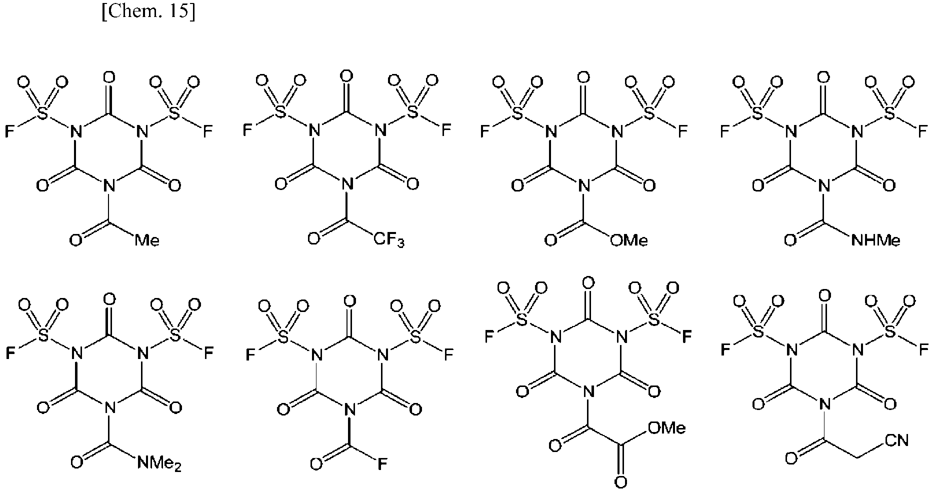

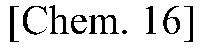

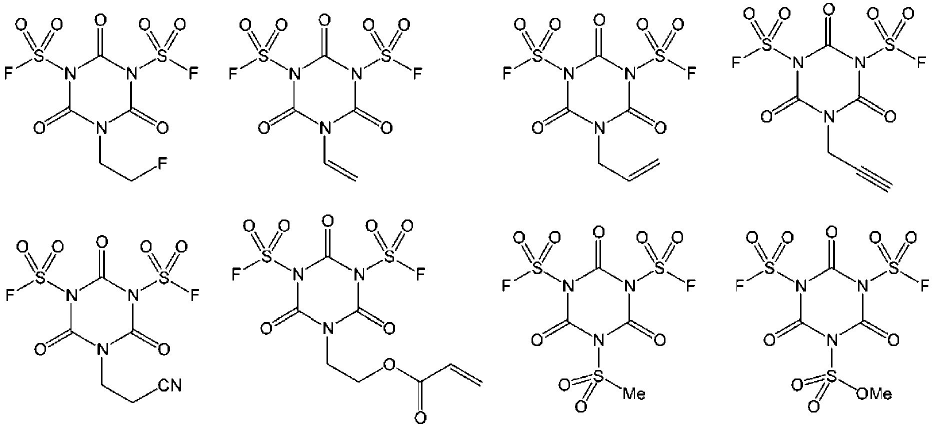

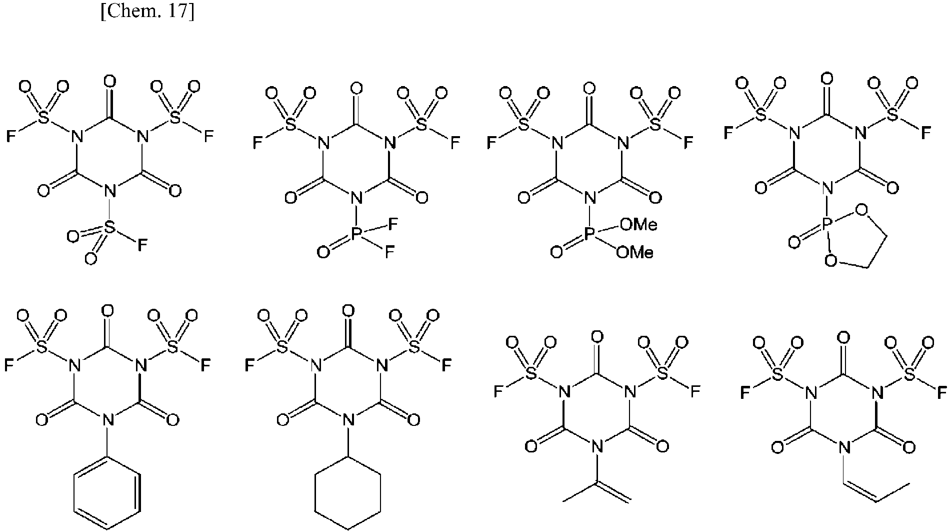

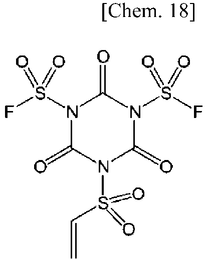

Of the specific examples shown above, at least one selected from the group consisting of the following compounds may be used.

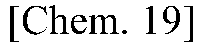

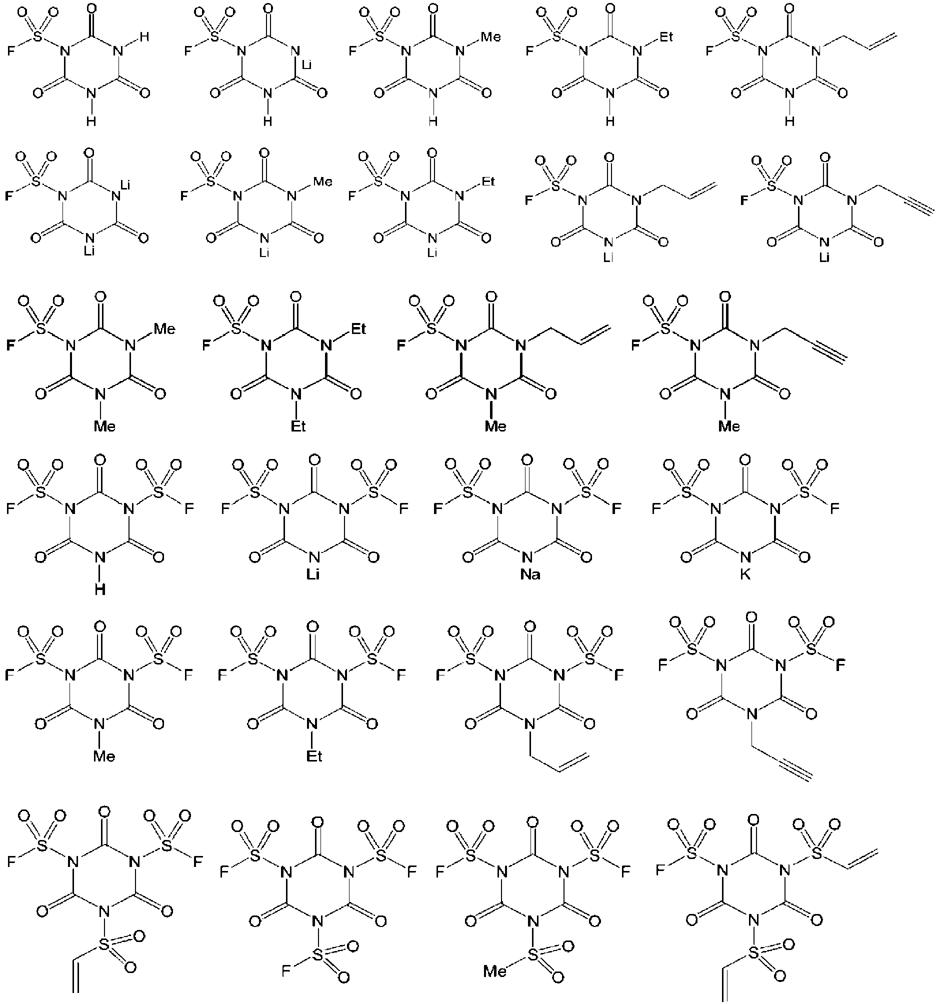

Moreover, of the specific examples shown above, in particular, at least one selected from the group consisting of the following compounds may be used.

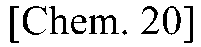

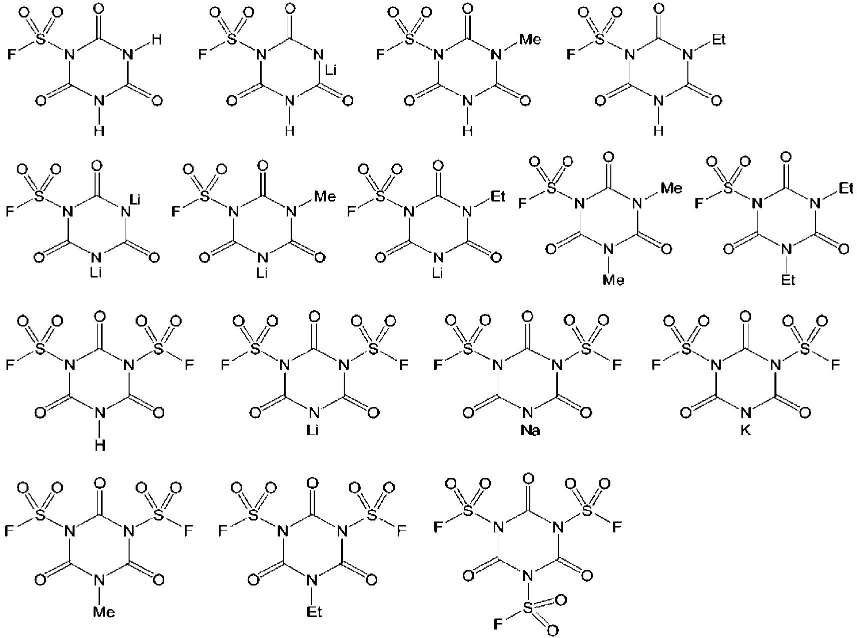

The compound represented by the general formula (2) is described below.

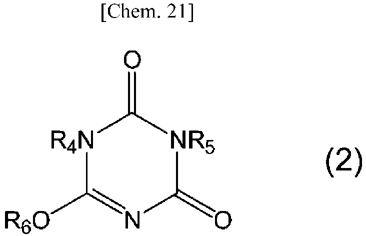

In the general formula (2), R₄ to R₆ represent groups which may be the same or different. Here, at least one of R₄ to R₆ represents a -SO₂F group.

Examples of the groups represented by R₄ to R₆ include a hydrogen atom, an alkali metal cation, a halogen atom, an alkyl group, an alkenyl group, an alkynyl group, a cycloalkyl group, a cycloalkenyl group, an aryl group, - S(=O)₂-R^{a}, -P(=O)-R^{b}R^{c}, -Si-R^{d}R^{e}R^{f}, and -C(=O)-R^{g}. R^{a} to R^{g} each independently represent a halogen atom, or an organic group selected from the group consisting of an alkyl group, an alkenyl group, an alkynyl group, a cycloalkyl group, a cycloalkenyl group, an aryl group, an alkoxy group, an alkenyloxy group, an alkynyloxy group, a cycloalkoxy group, a cycloalkenyloxy group, an aryloxy group, a monoalkylamino group, a dialkylamino group, and an alkoxycarbonyl group, and the organic group can contain at least one of an oxygen atom and an unsaturated bond. Moreover, R^{b} and R^{c} can bond to each other to form a cyclic structure.

When R₄ or R₅ represents an alkali metal cation, the bond between the nitrogen atom in the general formula (2) and R₄ or R₅ represents an ionic bond. When R₆ represents an alkali metal cation, the bond between the oxygen atom in the general formula (2) and R₆ represents an ionic bond.

In the groups represented by R₄ to R₆, at least any one hydrogen atom may be substituted with a substituent such as a halogen atom, a cyano group, and an acryloyloxy group. Examples of the halogen atom include a fluorine atom, a bromine atom, an iodine atom, and the like, and the halogen atom may be a fluorine atom.

Examples of the alkali metal cation, the halogen atom, the alkyl group, the alkenyl group, the alkynyl group, the cycloalkyl group, the cycloalkenyl group, and the aryl group represented by R₄ to R₆ include the alkali metal cation, the halogen atom, the alkyl group, the alkenyl group, the alkynyl group, the cycloalkyl group, the cycloalkenyl group, and the aryl group represented by R₁ to R₃ described above, and specific examples thereof are also the same.

Examples of R^{a} to R^{g} in the groups -S(=O)₂-R^{a}, -P(=O)-R^{b}R^{c}, -Si-R^{d}R^{e}R^{f}, and -C(=O)-R^{g} represented by R₄ to R₆ include R^{a} to R^{g} in the groups -S(=O)₂-R^{a}, -P(=O)-R^{b}R^{c}, -Si-R^{d}R^{e}R^{f}, and -C(=O)-R^{g} represented by R₁ to R₃ described above, and specific examples thereof are also the same.

At least one of R₄ to R₆ represents a -SO₂F group, and one or two thereof may be a -SO₂F group.

When a plurality of groups represented by R₄ to R₆ are other than a -SO₂F group, the groups may be each independently a group selected from the group consisting of a hydrogen atom, an alkali metal cation, an alkyl group, an alkenyl group, and an alkynyl group.

In particular, of R₄ to R₆, two may represent a -SO₂F group, and one may be a hydrogen atom, an alkali metal cation, an alkyl group, an alkenyl group, or an alkynyl group. Moreover, two thereof may represent a -SO₂F group, and one thereof may be an alkali metal cation.

When the compound represented by the general formula (2) has one -SO₂F group, R₆ may be the -SO₂F group.

Moreover, when the compound represented by the general formula (2) has two -SO₂F groups, R₄ and R₆ or R₅ and R₆ may be the -SO₂F groups.

From the viewpoint of stability of the compound, the compound represented by the general formula (2) may be a compound in which R₆ is a -SO₂F group.

Moreover, R₄ to R₆ may be all a -SO₂F group.

Specific examples of the compound represented by the general formula (2) are shown below, but the present invention is not limited thereto. In the following structural formulae, Me represents a methyl group, and Et represents an ethyl group.

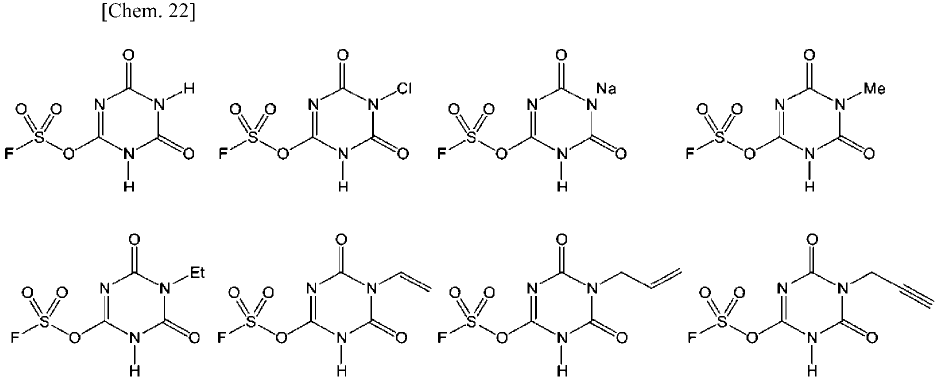

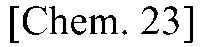

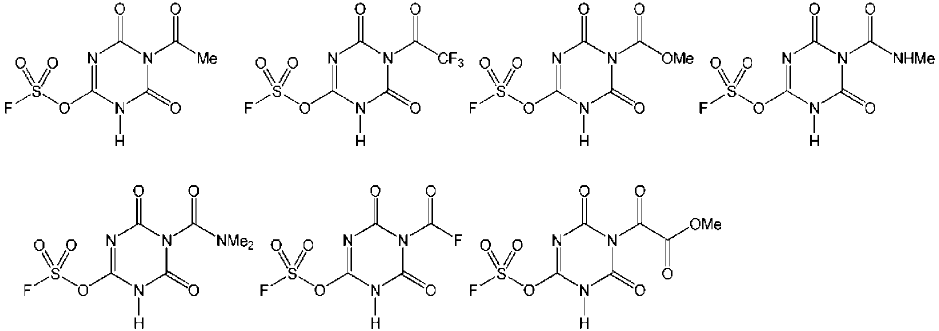

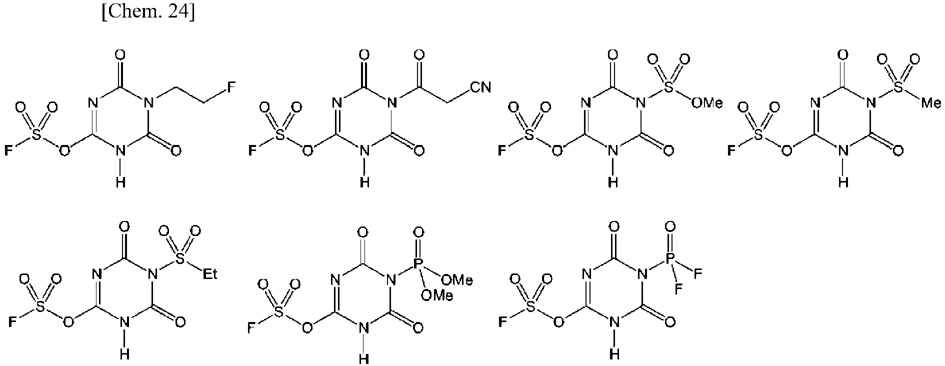

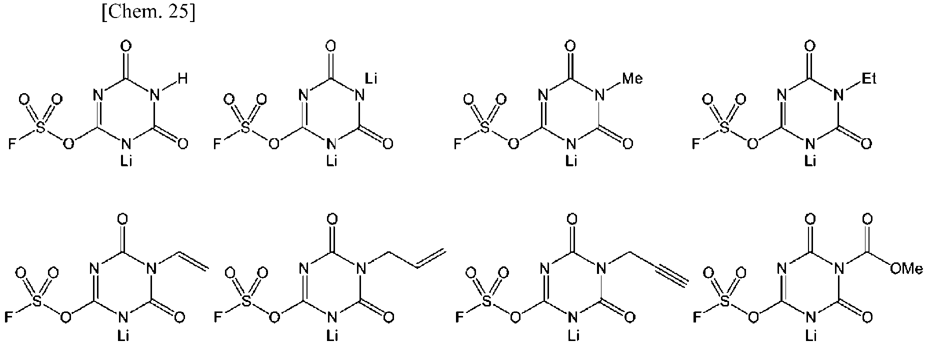

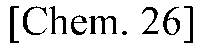

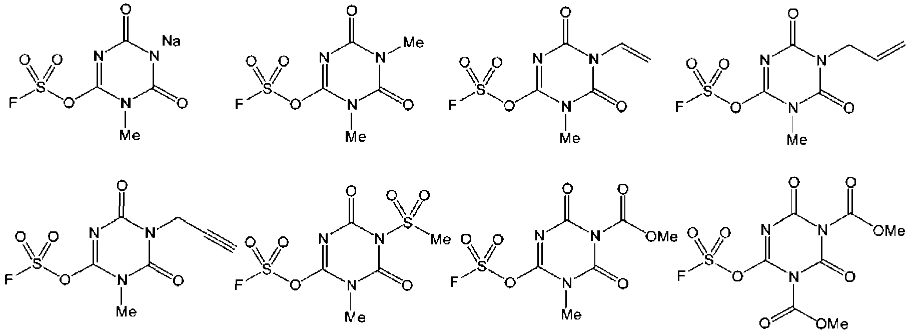

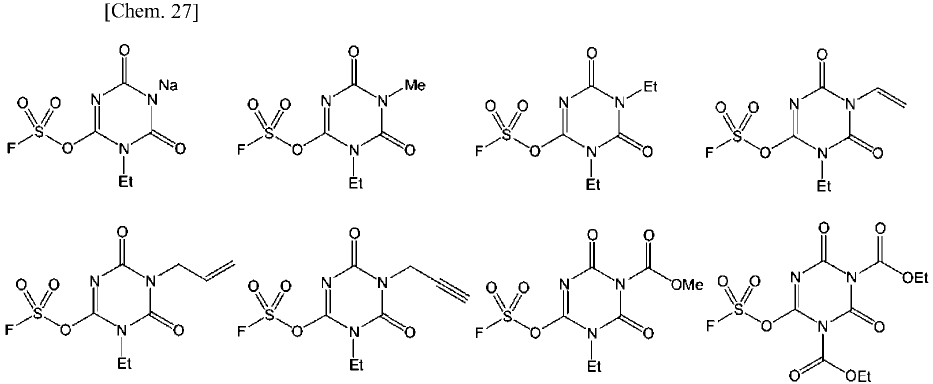

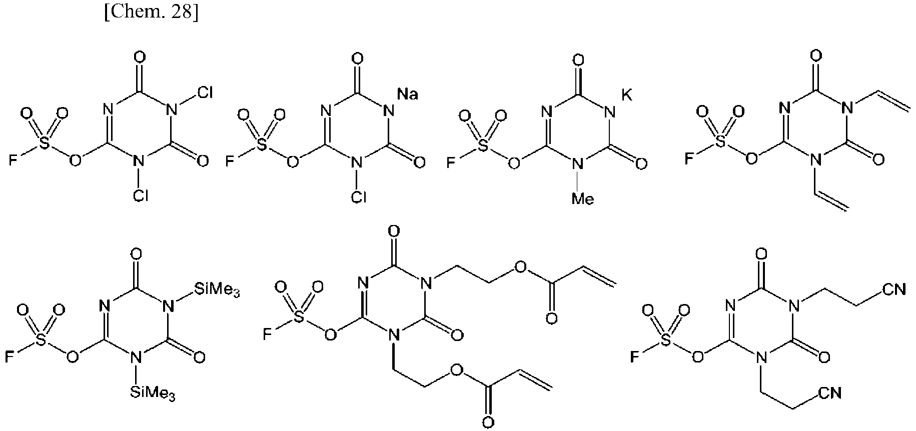

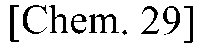

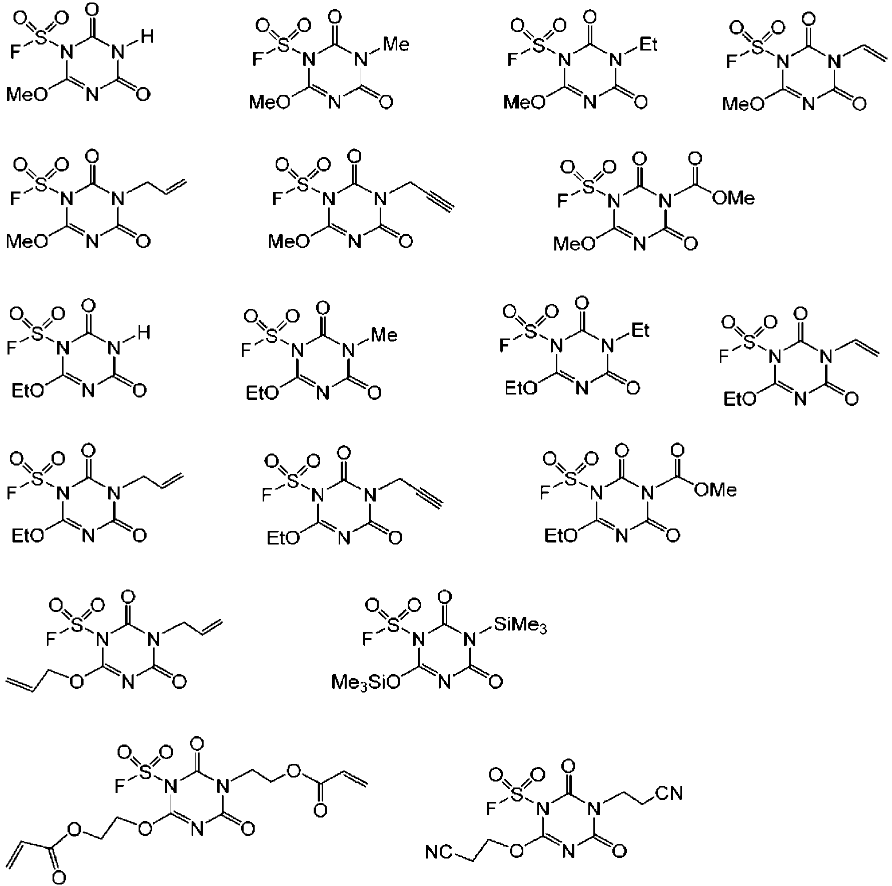

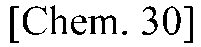

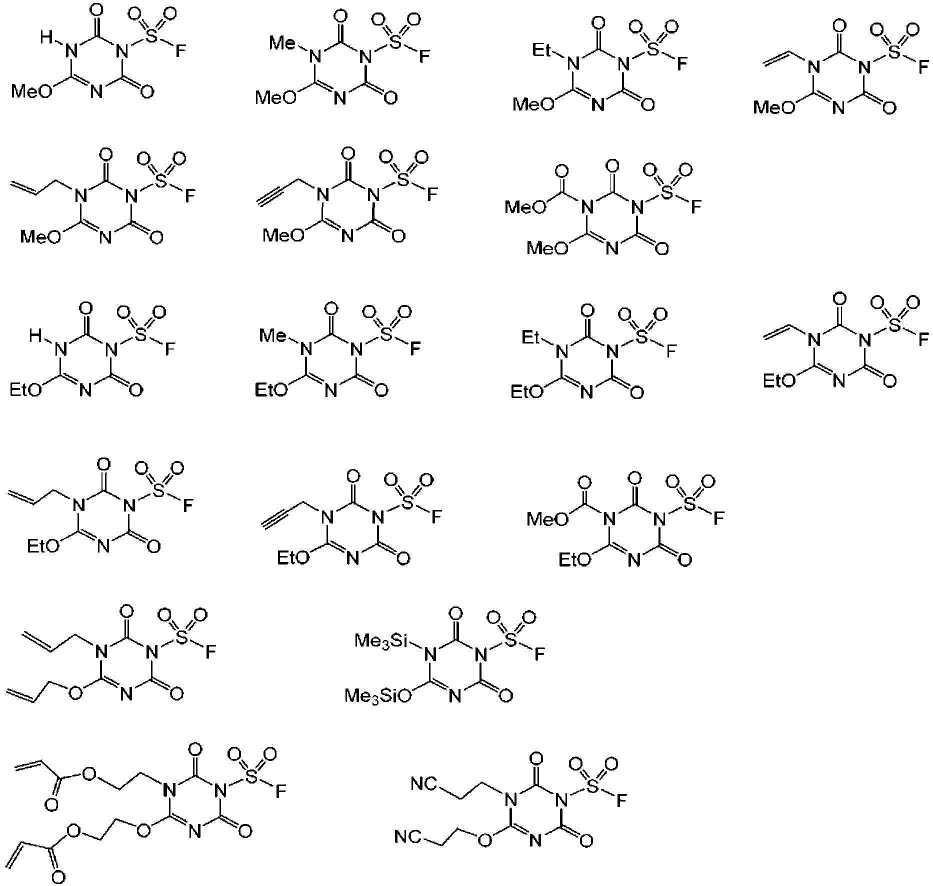

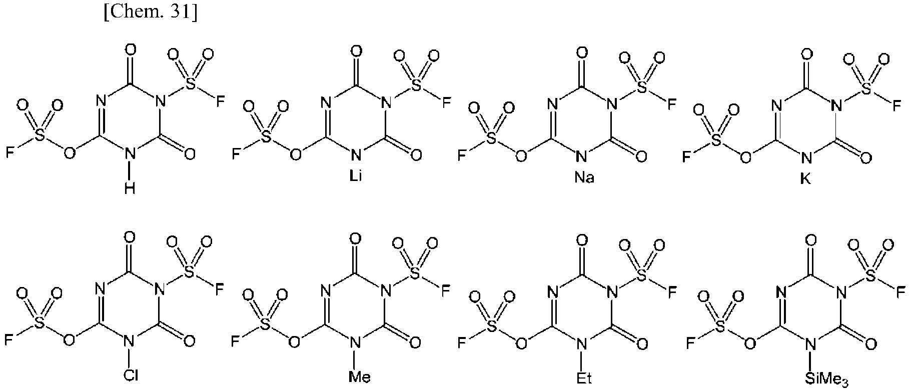

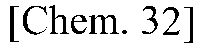

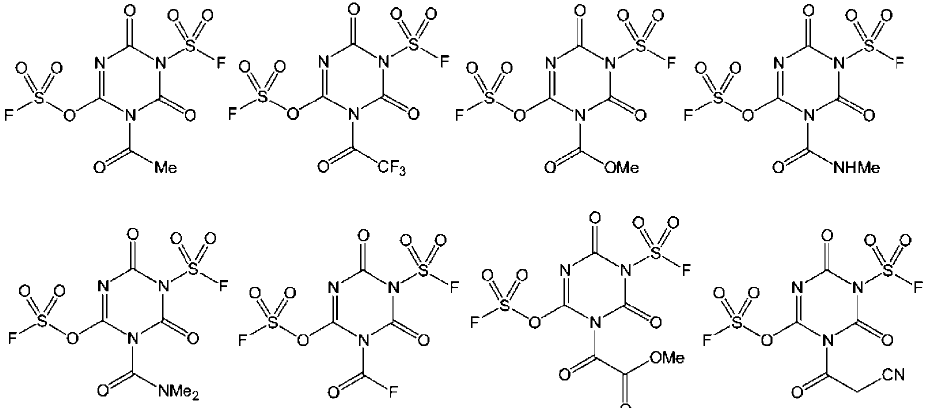

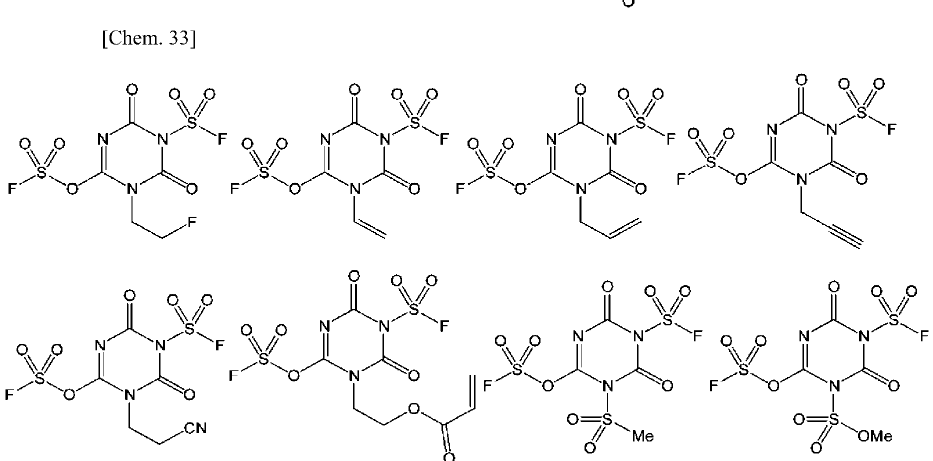

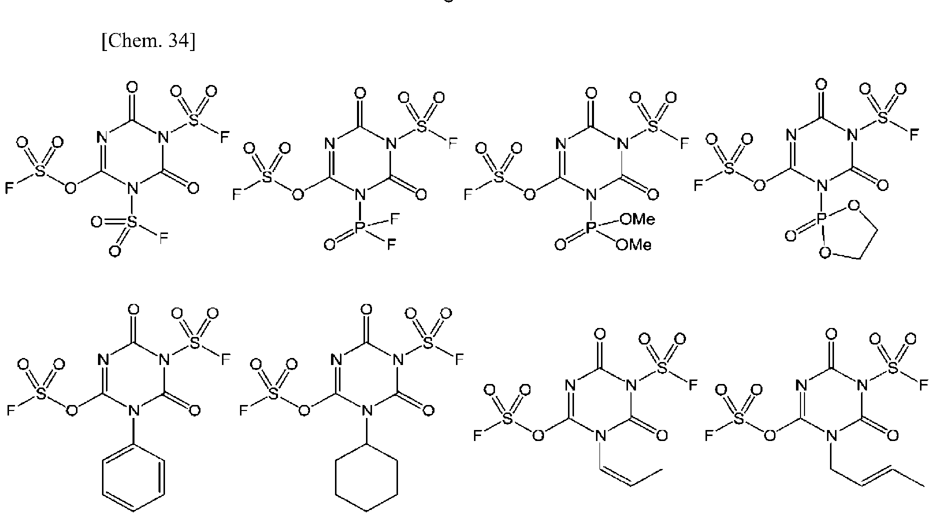

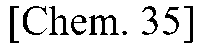

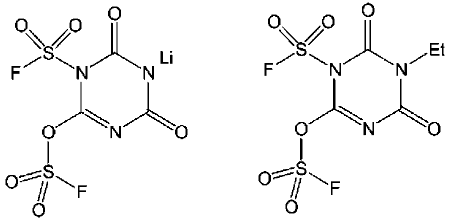

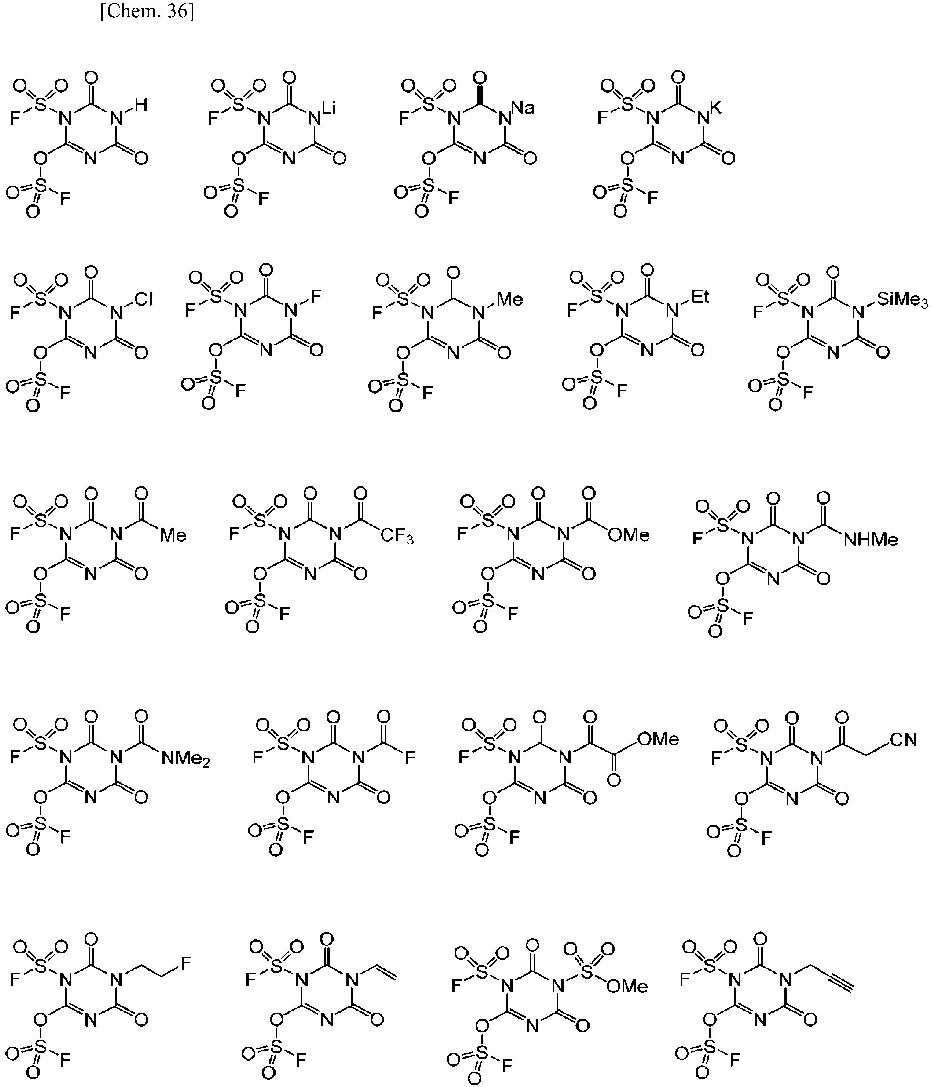

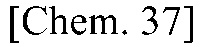

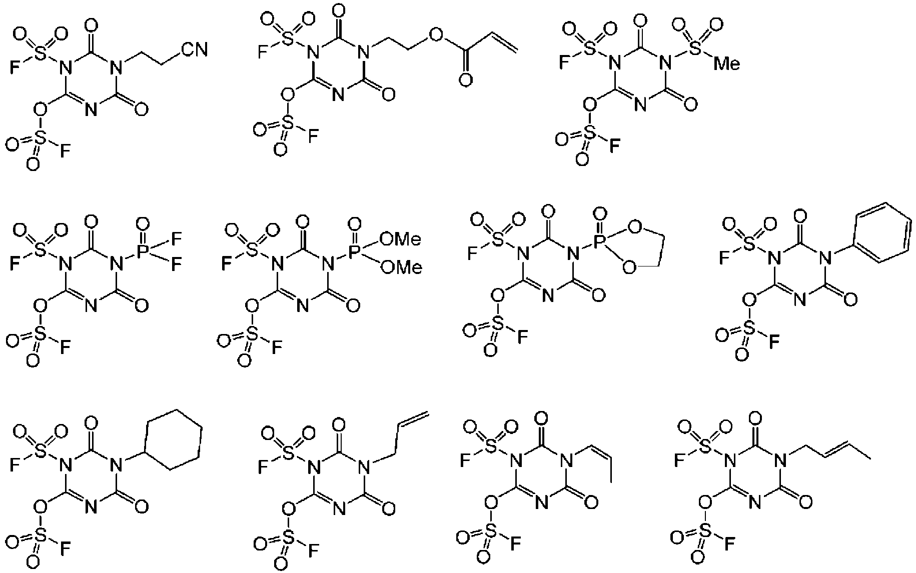

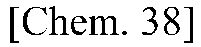

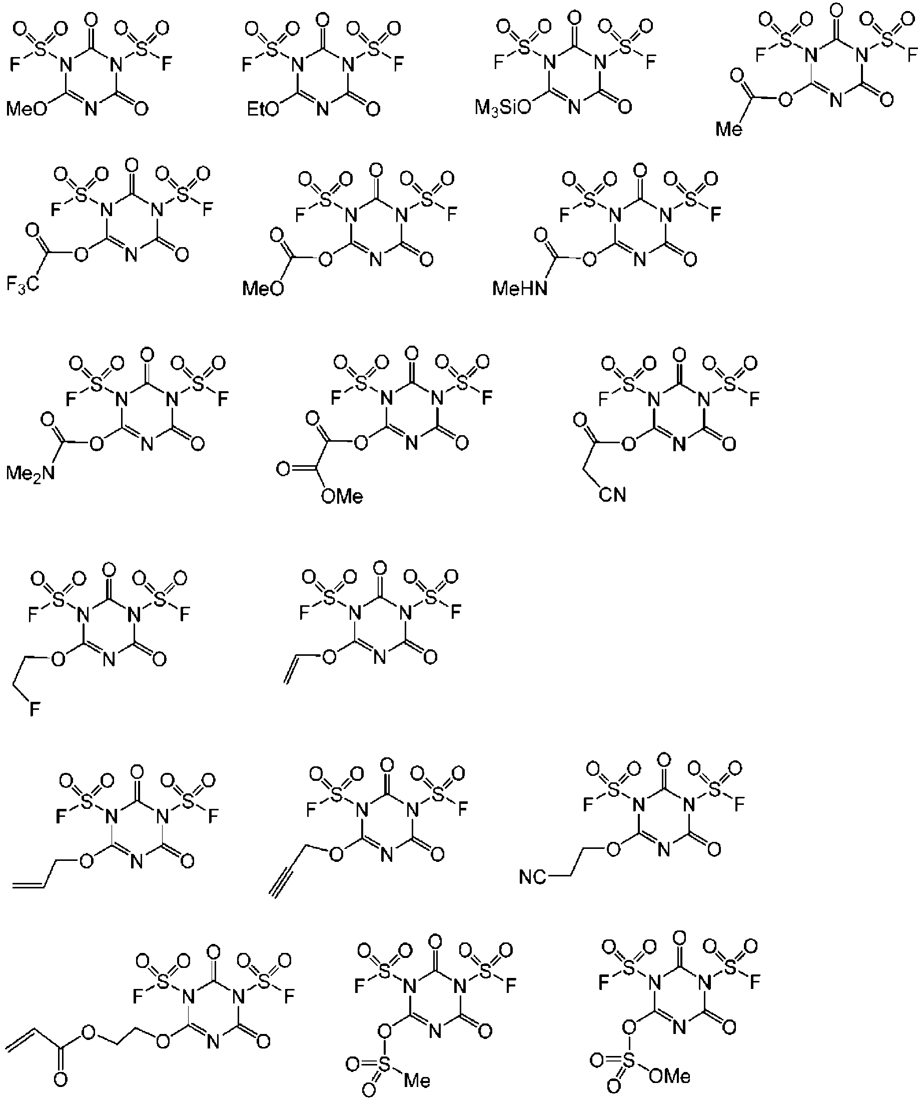

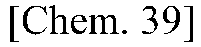

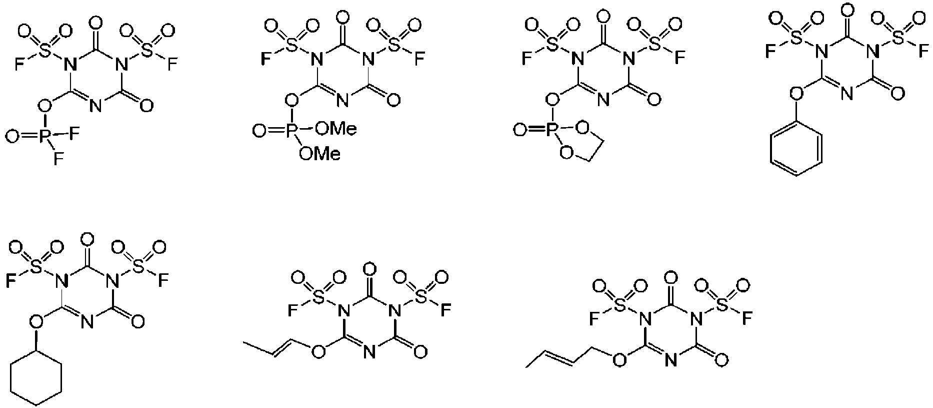

Of the specific examples shown above, at least one selected from the group consisting of the following compounds may be used.

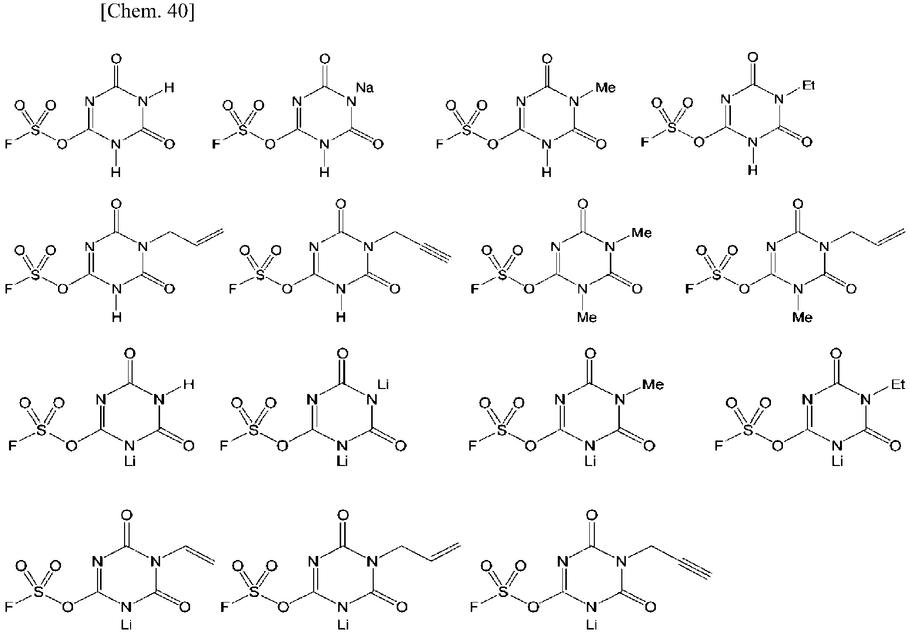

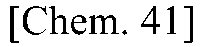

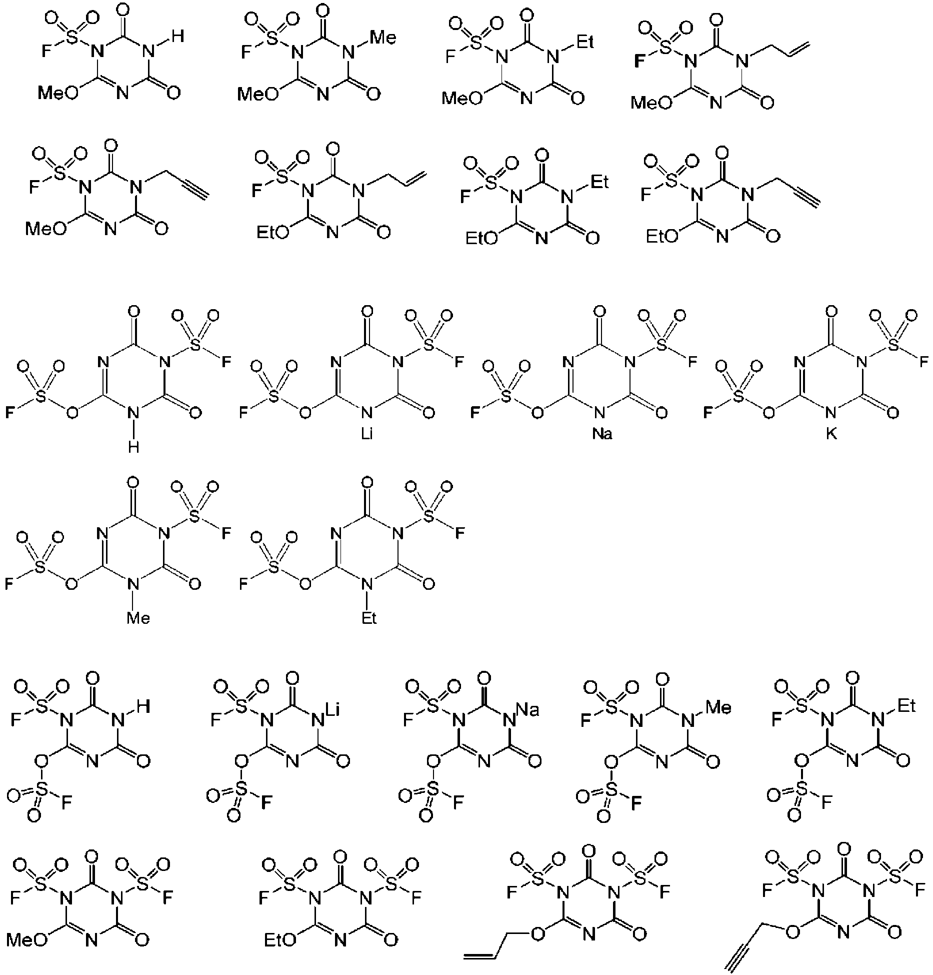

Moreover, of the specific examples shown above, in particular, at least one selected from the group consisting of the following compounds may be used.

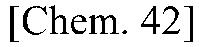

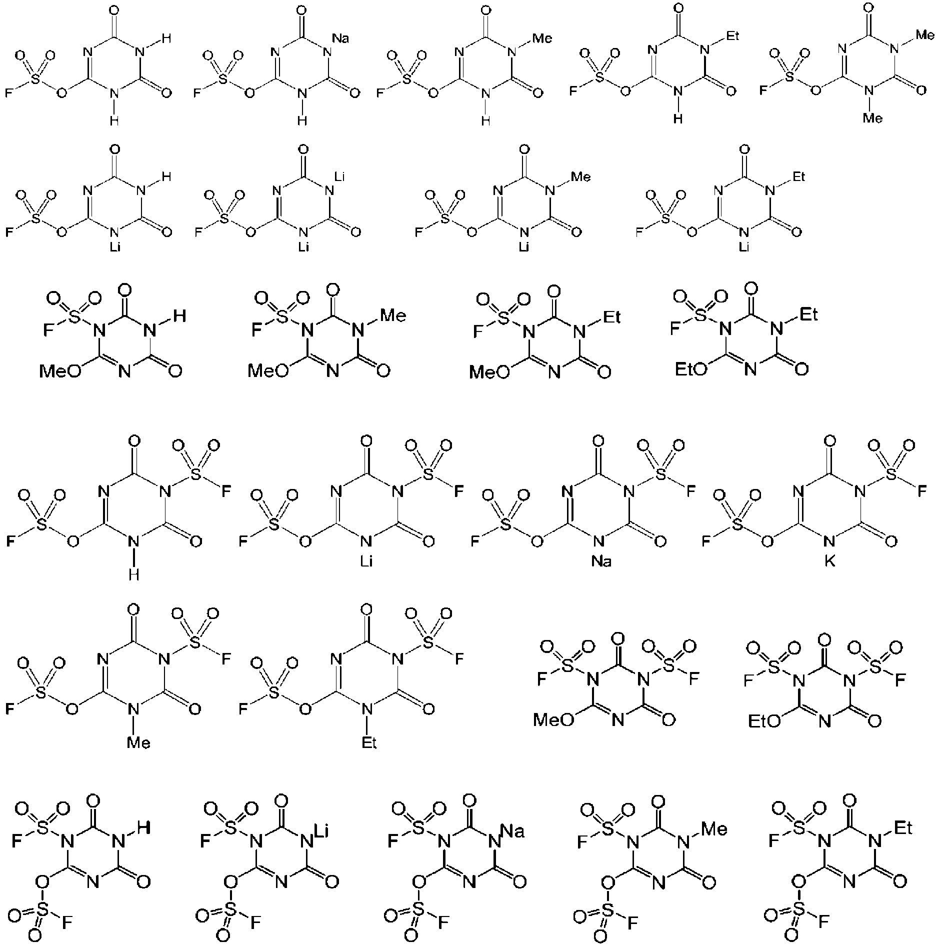

In the nonaqueous electrolyte solution of the present disclosure, the lower limit of the total amount of the component (I) with respect to the total amount (100% by mass) of the nonaqueous electrolyte solution (hereinafter also referred to as "concentration of (I)") may be 0.01% by mass or more, 0.08% by mass or more, or 0.3% by mass or more. The upper limit of the concentration of (I) may be 5.0% by mass or less, 2.5% by mass or less, or 1.8% by mass or less.

By setting the concentration of (I) to 0.01% by mass or more, an effect of preventing an increase in the initial resistance of the nonaqueous electrolyte solution battery using the nonaqueous electrolyte solution is easily obtained. On the other hand, by setting the concentration of (I) to 5.0% by mass or less, an increase in the viscosity of the nonaqueous electrolyte solution is easily prevented, and an effect of preventing an increase in the resistance of the nonaqueous electrolyte solution battery using the nonaqueous electrolyte solution is easily obtained.

In the nonaqueous electrolyte solution of the present disclosure, one type of compound may be used alone as the component (I), or two or more types of compounds may be mixed and used in any combination and any ratio according to an application.

The compound represented by the general formula (1) can be produced by various methods. The production method is not particularly limited.

For example, a compound in which R₁ to R₃ in the general formula (1) are all a -SO₂F group or a compound in which one or two of R₁ to R₃ are a -SO₂F group and the remainder is a hydrogen atom can be obtained by reacting isocyanuric acid and sulfuryl fluoride in the presence of a base.

The compound represented by the general formula (2) can be produced by various methods. The production method is not particularly limited.

For example, a compound in which one or two of R₄ to R₆ in the general formula (2) are a -SO₂F group and the remainder is an alkyl group can be obtained by reacting an alkyl isocyanate and fluorosulfonyl isocyanate in the presence of an amine catalyst.

The present disclosure also relates to the compound represented by the general formula (11) and the compound represented by the general formula (2).

The above compounds are suitably used as an additive in a nonaqueous electrolyte solution.

The nonaqueous electrolyte solution of the present disclosure may contain a solute (hereinafter sometimes referred to as "component (II)") and a nonaqueous organic solvent (hereinafter sometimes referred to as "component (III)") in addition to the component (I).

### <(II) Solute>

The nonaqueous electrolyte solution of the present disclosure may contain a solute.

The solute is not particularly limited but may be an ionic salt or an ionic salt containing fluorine.

The solute may be, for example, an ionic salt containing a pair of at least one cation selected from the group consisting of an alkali metal ion such as a lithium ion and a sodium ion, an alkaline earth metal ion, and quaternary ammonium and at least one anion selected from the group consisting of a hexafluorophosphate anion, a tetrafluoroborate anion, a perchlorate anion, a hexafluoroarsenate anion, a hexafluoroantimonate anion, a trifluoromethanesulfonate anion, a bis(trifluoromethanesulfonyl)imide anion, a bis(pentafluoroethanesulfonyl)imide anion, a (trifluoromethanesulfonyl)(pentafluoroethanesulfonyl)imide anion, a bis(fluorosulfonyl)imide anion, a (trifluoromethanesulfonyl)(fluorosulfonyl)imide anion, a (pentafluoroethanesulfonyl)(fluorosulfonyl)imide anion, and a tris(trifluoromethanesulfonyl)methide anion.

The solute may be at least one selected from the group consisting of LiPF₆, LiBF₄, LiSbF₆, LiAsF₆, LiClO₄, LiCF₃SO₃, LiC₄F₉SO₃, LiN(SO₂F)₂, LiAlO₂, LiAlCl₄, LiCl, and LiI or at least one selected from the group consisting of NaPF₆, NaBF₄, NaSbF₆, NaAsF₆, NaClO₄, NaCF₃SO₃, NaC₄F₉SO₃, NaN(SO₂F)₂, NaAlO₂, NaAlCl₄, NaCl, and Nal.

One type of these solutes may be used alone, or two or more types thereof may be mixed and used in any combination and any ratio according to an application.

Of the solutes, considering the energy density, the output characteristic, the life, and the like of the nonaqueous electrolyte solution battery, the cation may be at least one selected from the group consisting of lithium, sodium, potassium, magnesium, and quaternary ammonium, and the anion may be at least one selected from the group consisting of a hexafluorophosphate anion, a tetrafluoroborate anion, a bis(trifluoromethanesulfonyl)imide anion, and a bis(fluorosulfonyl)imide anion.

The total amount of the solute in the nonaqueous electrolyte solution of the present disclosure (hereinafter also referred to as "solute concentration") is not particularly restricted, but the lower limit may be 0.5 mol/L or more, 0.7 mol/L or more, or 0.9 mol/L or more. The upper limit of the solute concentration may be 5.0 mol/L or less, 4.0 mol/L or less, or 2.0 mol/L or less. By setting the solute concentration to 0.5 mol/L or more, the decrease in the cycle characteristic and the output characteristic of the nonaqueous electrolyte solution battery, due to a decrease in the ionic conductivity, is easily prevented, and by setting the solute concentration to 5.0 mol/L or less, the decrease in the ionic conductivity and the decrease in the cycle characteristic and the output characteristic of the nonaqueous electrolyte solution battery, due to an increase in the viscosity of the nonaqueous electrolyte solution, is easily prevented.

### <(III) Nonaqueous Organic Solvent>

The nonaqueous electrolyte solution of the present disclosure may contain a nonaqueous organic solvent. The type of the nonaqueous organic solvent is not particularly limited, and any nonaqueous organic solvent can be used.

The nonaqueous organic solvent may contain at least one selected from the group consisting of a cyclic ester, a chain ester, a cyclic ether, a chain ether, a sulfone compound, a sulfoxide compound, and an ionic liquid.

Specifically, the nonaqueous organic solvent may contain at least one selected from the group consisting of ethyl methyl carbonate (hereinafter also referred to as "EMC"), dimethyl carbonate (hereinafter also referred to as "DMC"), diethyl carbonate (hereinafter also referred to as "DEC"), methyl propyl carbonate, ethyl propyl carbonate, methyl butyl carbonate, methyl 2,2,2-trifluoroethyl carbonate, ethyl 2,2,2-trifluoroethyl carbonate, propyl 2,2,2-trifluoroethyl carbonate, bis(2,2,2-trifluoroethyl) carbonate, 1,1,1,3,3,3-hexafluoro-1-propylmethyl carbonate, 1,1,1,3,3,3-hexafluoro-1-propylethyl carbonate, 1,1,1,3,3,3-hexafluoro-1-propylpropyl carbonate, bis(1,1,1,3,3,3-hexafluoro-1-propyl) carbonate, ethylene carbonate (hereinafter also referred to as "EC"), propylene carbonate (hereinafter also referred to as "PC"), butylene carbonate, fluoroethylene carbonate (hereinafter also referred to as "FEC"), difluoroethylene carbonate, methyl acetate, ethyl acetate, methyl propionate, ethyl propionate, methyl 2-fluoropropionate, ethyl 2-fluoropropionate, diethyl ether, dibutyl ether, diisopropyl ether, 1,2-dimethoxyethane, tetrahydrofuran, 2-methyltetrahydrofuran, furan, tetrahydropyran, 1,3-dioxane, 1,4-dioxane, N,N-dimethylformamide, acetonitrile, propionitrile, dimethyl sulfoxide, sulfolane, γ-butyrolactone, and γ-valerolactone.

In the present disclosure, an ionic liquid having a salt structure may be used as the nonaqueous organic solvent.

In terms of an excellent input/output characteristic at low temperature, at least one selected from the group consisting of a cyclic ester and a chain ester may be contained as the nonaqueous organic solvent in the nonaqueous electrolyte solution.

In addition, in terms of the excellent cycle characteristic at high temperature, at least one selected from the group consisting of a cyclic carbonate and a chain carbonate may be contained as the nonaqueous organic solvent in the nonaqueous electrolyte solution.

The nonaqueous organic solvent may contain a cyclic ester, and the cyclic ester may be a cyclic carbonate.

Specific examples of the cyclic carbonate include EC, PC, butylene carbonate, FEC, and the like, and of these, the cyclic carbonate may be at least one selected from the group consisting of EC, PC, and FEC.

The nonaqueous organic solvent may contain a chain ester, and the chain ester may be a chain carbonate.

Specific examples of the chain carbonate include EMC, DMC, DEC, methyl propyl carbonate, ethyl propyl carbonate, methyl 2,2,2-trifluoroethyl carbonate, ethyl 2,2,2-trifluoroethyl carbonate, 1,1,1,3,3,3-hexafluoro-1-propylmethyl carbonate, 1,1,1,3,3,3-hexafluoro-1-propylethyl carbonate, and the like, and of these, the chain carbonate may be at least one selected from the group consisting of EMC, DMC, DEC, and methyl propyl carbonate.

Specific examples of the chain ester include methyl acetate, ethyl acetate, methyl propionate, ethyl propionate, methyl 2-fluoropropionate, ethyl 2-fluoropropionate, methyl 3,3,3-trifluoropropionate, and the like.

### <Other Additive>

The nonaqueous electrolyte solution of the present disclosure may further contain an additive component which is generally used in any ratio as long as the gist of the present disclosure is not impaired.

Specific examples of the other additive include compounds having an overcharge prevention effect, a negative electrode film-forming effect, and a positive electrode protective effect, such as cyclohexylbenzene, cyclohexylfluorobenzene, fluorobenzene, biphenyl, difluoroanisole, tert-butylbenzene, tert-amylbenzene, 2-fluorotoluene, 2-fluorobiphenyl, vinylene carbonate, dimethylvinylene carbonate, vinylethylene carbonate, fluoroethylene carbonate, trans-difluoroethylene carbonate, methyl propargyl carbonate, ethyl propargyl carbonate, dipropargyl carbonate, maleic anhydride, succinic anhydride, propanesultone, 1,3-propanesultone, 1,3-propenesultone, butanesultone, 1,3,2-dioxathiolane-2,2-dioxide, 4-propyl-1,3,2-dioxathiolane-2,2-dioxide, 1,2-ethanedisulfonic anhydride, methylene methanedisulfonate, dimethylene methanedisulfonate, trimethylene methanedisulfonate, methyl methanesulfonate, 1,6-diisocyanatohexane, tris(trimethylsilyl)borate, succinonitrile, (ethoxy)pentafluorocyclotriphosphazene, lithium difluorobis(oxalato)phosphate, sodium difluorobis(oxalato)phosphate, potassium difluorobis(oxalato)phosphate, lithium difluorooxalatoborate, sodium difluorooxalatoborate, potassium difluorooxalatoborate, lithium bis(oxalato)borate, sodium bis(oxalato)borate, potassium bis(oxalato)borate, lithium tetrafluorooxalatophosphate, sodium tetrafluorooxalatophosphate, potassium tetrafluorooxalatophosphate, lithium tris(oxalato)phosphate, sodium tris(oxalato)phosphate, potassium tris(oxalato)phosphate, lithium difluorophosphate, sodium difluorophosphate, potassium difluorophosphate, lithium monofluorophosphate, sodium monofluorophosphate, potassium monofluorophosphate, lithium fluorosulfonate, sodium fluorosulfonate, potassium fluorosulfonate, lithium bis(difluorophosphoryl)imide, sodium bis(difluorophosphoryl)imide, potassium bis(difluorophosphoryl)imide, methanesulfonyl fluoride, ethenesulfonyl fluoride, and phenyl difluorophosphate.

The nonaqueous electrolyte solution of the present disclosure may contain at least one selected from vinylene carbonate, bis(oxalato)borate, difluorooxalatoborate, difluorobis(oxalato)phosphate, tetrafluorooxalatophosphate, (difluorophosphoryl)(fluorosulfonyl)imide salt, difluorophosphate, fluorosulfonate, 1,3-propenesultone, 1,3-propanesultone, 1,6-diisocyanatohexane, ethynylethylene carbonate, 1,3,2-dioxathiolane-2,2-dioxide, 4-propyl-1,3,2-dioxathiolane-2,2-dioxide, methylene methanedisulfonate, 1,2-ethanedisulfonic anhydride, methanesulfonyl fluoride, tris(trimethylsilyl)borate, (ethoxy)pentafluorocyclotriphosphazene, tetrafluoro(malonato)phosphate, tetrafluoro(picolinato)phosphate, 1,3-dimethyl-1,3-divinyl-1,3-di(1,1,1,3,3,3-hexafluoroisopropyl)disiloxane, tetravinylsilane, t-butylbenzene, t-amylbenzene, fluorobenzene, and cyclohexylbenzene.

The additive content of the nonaqueous electrolyte solution may be 0.01% by mass or more and 5.0% by mass or less with respect to the total amount of the nonaqueous electrolyte solution.

The nonaqueous electrolyte solution of the present disclosure may contain a compound represented by the following general formula (4) as the other additive.

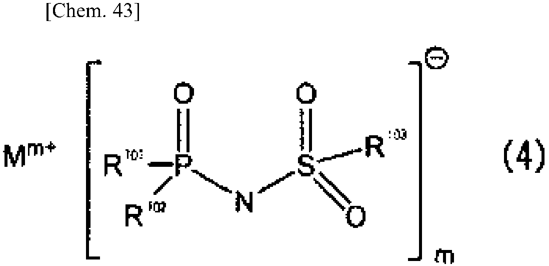

[In the general formula (4), R¹⁰¹ to R¹⁰³ are each independently a fluorine atom or an organic group selected from a linear alkyl group having 1 to 10 carbon atoms, a branched alkyl group having 3 to 10 carbon atoms, a linear alkoxy group having 1 to 10 carbon atoms, a branched alkoxy group having 3 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 10 carbon atoms, a cycloalkoxy group having 3 to 10 carbon atoms, a cycloalkenyl group having 3 to 10 carbon atoms, a cycloalkenyloxy group having 3 to 10 carbon atoms, an aryl group having 6 to 10 carbon atoms, and an aryloxy group having 6 to 10 carbon atoms, and the organic group can have a fluorine atom, an oxygen atom, or an unsaturated bond. Here, at least one of R¹⁰¹ to R¹⁰³ is a fluorine atom.

M^{m+} is an alkali metal cation, an alkaline earth metal cation, or an onium cation, and m represents the same integer as the valence of the corresponding cation.]

When the compound represented by the general formula (4) (a salt having an imide anion) has at least one P-F bond or S-F bond, excellent low-temperature characteristic is obtained. As the number of the P-F bonds and the S-F bonds in the salt having the imide anion is larger, the low-temperature characteristic can be further improved, and thus the salt having the imide anion represented by the general formula (4) may be a compound in which all of R¹⁰¹ to R¹⁰³ are fluorine atoms.

Moreover, the salt having the imide anion represented by the general formula (4) may be a compound in which at least one of R¹⁰¹ to R¹⁰³ is a fluorine atom and at least one of R¹⁰¹ to R¹⁰³ is selected from a hydrocarbon group having 6 or less carbon atoms which may have a fluorine atom.

In addition, the salt having the imide anion represented by the general formula (4) may be a compound in which at least one of R¹⁰¹ to R¹⁰³ is a fluorine atom and at least one of R¹⁰¹ to R¹⁰³ is selected from a methyl group, a methoxy group, an ethyl group, an ethoxy group, a propyl group, a propoxy group, a vinyl group, an allyl group, an allyloxy group, an ethynyl group, a 2-propynyl group, a 2-propynyloxy group, a phenyl group, a phenyloxy group, a 2,2-difluoroethyl group, a 2,2-difluoroethyloxy group, a 2,2,2-trifluoroethyl group, a 2,2,2-trifluoroethyloxy group, a 2,2,3,3-tetrafluoropropyl group, a 2,2,3,3-tetrafluoropropyloxy group, a 1,1,1,3,3,3-hexafluoroisopropyl group, and a 1,1,1,3,3,3-hexafluoroisopropyloxy group.

The counter cation M^{m+} of the salt having the imide anion represented by the general formula (4) may be selected from the group consisting of a lithium ion, a sodium ion, a potassium ion, and a tetraalkylammonium ion.

In the general formula (4), examples of the alkyl group and the alkoxy group represented by R¹⁰¹ to R¹⁰³ include an alkyl group having 1 to 10 carbon atoms and a fluorine-containing alkyl group, such as a methyl group, an ethyl group, a propyl group, an isopropyl group, a butyl group, a sec-butyl group, a tert-butyl group, a pentyl group, a 2,2-difluoroethyl group, 2,2,2-trifluoroethyl group, 2,2,3,3-tetrafluoropropyl group, and a 1,1,1,3,3,3-hexafluoroisopropyl group, and an alkoxy group derived from these groups.

Examples of the alkenyl group and the alkenyloxy group include an alkenyl group having 2 to 10 carbon atoms such as a vinyl group, an allyl group, a I-propenyl group, an isopropenyl group, a 2-butenyl group, and a 1,3-butadienyl group, a fluorine-containing alkenyl group, and an alkenyloxy group derived from these groups.

Examples of the alkynyl group and the alkynyloxy group include an alkynyl group having 2 to 10 carbon atoms such as an ethynyl group, a 2-propynyl group, and a 1,1-dimethyl-2-propynyl group, a fluorine-containing alkynyl group, and an alkynyloxy group derived from these groups.

Examples of the cycloalkyl group and the cycloalkoxy group include a cycloalkyl group having 3 to 10 carbon atoms such as a cyclopentyl group and a cyclohexyl group, a fluorine-containing cycloalkyl group, and a cycloalkoxy group derived from these groups.

Examples of the cycloalkenyl group and the cycloalkenyloxy group include a cycloalkenyl group having 3 to 10 carbon atoms such as a cyclopentenyl group and a cyclohexenyl group, a fluorine-containing cycloalkenyl group, and a cycloalkenyloxy group derived from these groups.

Examples of the aryl group and the aryloxy group include an aryl group having 6 to 10 carbon atoms such as a phenyl group, a tolyl group, and a xylyl group, a fluorine-containing aryl group, and an aryloxy group derived from these groups.

Specific examples and synthesis methods of the salt having the imide anion represented by the general formula (4) include those described in WO2017/111143.

The other additive content of the nonaqueous electrolyte solution may be 0.01% by mass or more and 8.0% by mass or less with respect to the total amount of the nonaqueous electrolyte solution.

When the content of the ionic salt exemplified as the solute in the nonaqueous electrolyte solution is less than 0.5 mol/L, which is the lower limit of the suitable concentration of the solute, the ionic salt can exert a negative electrode film-forming effect and a positive electrode protective effect as the "other additive". In this case, the content in the nonaqueous electrolyte solution is preferably 0.01% by mass to 5.0% by mass.

For example, when the nonaqueous electrolyte solution battery is a lithium ion battery, examples of the ionic salt in this case include lithium hexafluorophosphate, lithium tetrafluoroborate, lithium trifluoromethanesulfonate, lithium bis(trifluoromethanesulfonyl)imide, lithium bis(fluorosulfonyl)imide, lithium (trifluoromethanesulfonyl)(fluorosulfonyl)imide, and the like, and when the nonaqueous electrolyte solution battery is a sodium ion battery, examples of the ionic salt include sodium hexafluorophosphate, sodium tetrafluoroborate, sodium trifluoromethanesulfonate, sodium bis(trifluoromethanesulfonyl)imide, sodium bis(fluorosulfonyl)imide, sodium (trifluoromethanesulfonyl)(fluorosulfonyl)imide, and the like.

An alkali metal salt other than the solutes may be used as an additive.

Specific examples include carboxylates such as lithium acrylate, sodium acrylate, lithium methacrylate, and sodium methacrylate, sulfate ester salts such as lithium methyl sulfate, sodium methyl sulfate, lithium ethyl sulfate, and sodium ethyl sulfate, and the like.

From the viewpoint of improving the durability (life) of the battery, when the nonaqueous electrolyte solution battery is a lithium ion battery, the nonaqueous electrolyte solution of the present disclosure may contain, with respect to the total amount of the nonaqueous electrolyte solution, 0.01 to 5.0% by mass of at least one selected from vinylene carbonate, fluoroethylene carbonate, lithium bis(oxalato)borate, lithium difluorooxalatoborate, lithium difluorobis(oxalato)phosphate, lithium tetrafluorooxalatophosphate, lithium bis(fluorosulfonyl)imide, lithium (difluorophosphoryl)(fluorosulfonyl)imide, lithium difluorophosphate, lithium fluorosulfonate, 1,3-propenesultone, 1,3-propanesultone, 1,3,2-dioxathiolane-2,2-dioxide, 1,2-ethanedisulfonic anhydride, and 4-propyl-1,3,2-dioxathiolane-2,2-dioxide, of the other additives.

From the same viewpoint, when the nonaqueous electrolyte solution battery is a sodium ion battery, the nonaqueous electrolyte solution may contain, with respect to the total amount of the nonaqueous electrolyte solution, 0.01 to 5.0% by mass of at least one selected from vinylene carbonate, fluoroethylene carbonate, sodium bis(oxalato)borate, sodium difluorooxalatoborate, sodium difluorobis(oxalato)phosphate, sodium tetrafluorooxalatophosphate, sodium bis(fluorosulfonyl)imide, sodium (difluorophosphoryl)(fluorosulfonyl)imide, sodium difluorophosphate, sodium fluorosulfonate, 1,3-propenesultone, 1,3-propanesultone, 1,3,2-dioxathiolane-2,2-dioxide, 1,2-ethanedisulfonic anhydride, and 4-propyl-1,3,2-dioxathiolane-2,2-dioxide.

In addition, the nonaqueous electrolyte solution of the present disclosure can also contain a polymer, and as in the case of being used in a nonaqueous electrolyte solution battery referred to as a polymer battery, the nonaqueous electrolyte solution can be quasi-solidified with a gelling agent or a cross-linked polymer before use. The polymer solid electrolyte includes one containing a nonaqueous organic solvent as a plasticizer.

The above polymer is not particularly limited as long as the polymer is an aprotic polymer capable of dissolving the compound represented by the general formula (1) or (2), the solute, and the other additive. Examples thereof include polymers having polyethylene oxide as a principal chain or a side chain, homopolymers or copolymers of polyvinylidene fluoride, methacrylic acid ester polymers, polyacrylonitrile, and the like. When a plasticizer is added to such a polymer, of the above nonaqueous organic solvents, an aprotic nonaqueous organic solvent may be used.

### [2. Nonaqueous Electrolyte Solution Battery]

The nonaqueous electrolyte solution battery of the present disclosure includes at least the nonaqueous electrolyte solution of the present disclosure described above, a negative electrode, and a positive electrode. Further, a separator, an exterior body, and the like may be included.

The nonaqueous electrolyte solution battery of the present disclosure preferably includes at least a positive electrode, a negative electrode, a separator, and the nonaqueous electrolyte solution of the present disclosure.

The nonaqueous electrolyte solution battery of the present disclosure is preferably a nonaqueous electrolyte solution secondary battery.

The negative electrode is not particularly limited, but a material capable of reversibly intercalating and deintercalating alkali metal ions such as lithium ions and sodium ions or alkaline earth metal ions may be used.

For example, in the case of a lithium ion secondary battery in which cations are mainly lithium, the negative electrode active material constituting the negative electrode is one capable of doping and dedoping lithium ions, and examples thereof include those containing at least one selected from a carbon material in which the d value of the lattice plane (002 plane) in X-ray diffraction is 0.340 nm or less, a carbon material in which the d value of the lattice plane (002 plane) in X-ray diffraction exceeds 0.340 nm, oxides of one or more metals selected from Si, Sn, and Al, one or more metals selected from Si, Sn, and Al, alloys containing these metals, alloys of the metals or the alloys and lithium, and lithium titanium oxide. One type of these negative electrode active materials can be used alone, or two or more types thereof can be used in combination. Lithium metal, a metal nitride, a tin compound, a conductive polymer, and the like may also be used.

For example, in the case of a sodium ion secondary battery in which the cations are mainly sodium, as the negative electrode active material constituting the negative electrode, sodium metal, an alloy of sodium metal and another metal such as tin, an intermetallic compound, various carbon materials such as hard carbon, a metal oxide such as titanium oxide, a metal nitride, (elemental) tin, a tin compound, activated carbon, a conductive polymer, and the like may be used. In addition to these, (elemental) phosphorus such as red phosphorus and black phosphorus, phosphorus compounds such as Co-P, Cu-P, Sn-P, Ge-P, and Mo-P, (elemental) antimony, antimony compounds such as Sb/C and Bi-Sb, and the like may be used. One type of these negative electrode active materials may be used alone, or two or more types thereof may be used in combination.

The positive electrode is not particularly limited, but a material capable of reversibly intercalating and deintercalating alkali metal ions such as lithium ions and sodium ions or alkaline earth metal ions may be used.

For example, when the cation is lithium, lithium-containing transition metal oxide composites such as LiCoO₂, LiNiO₂, LiMnO₂, and LiMn₂O₄, lithium-containing transition metal oxide composites including a mixture of a plurality of transition metals in the lithium-containing transition metal oxide composites such as Co, Mn, and Ni, lithium-containing transition metal oxide composites in which a part of the transition metals in the lithium-containing transition metal oxide composites is substituted with a metal other than the transition metals, phosphate compounds of transition metals such as LiFePO₄, LiCoPO₄, and LiMnPO₄ referred to as olivine, oxides such as TiO₂, V₂O₅, and MoO₃, sulfides such as TiS₂ and FeS, conductive polymers such as polyacetylene, polyparaphenylene, polyaniline, and polypyrrole, activated carbon, radical-generating polymers, carbon materials, and the like may be used as a positive electrode material.

For example, when the cation is sodium, sodium-containing transition metal oxide composites such as NaCrO₂, NaFe_{0.5}Co_{0.5}O₂, NaFe_{0.4}Mn_{0.3}Ni_{0.3}O₂, NaNi_{0.5}Ti_{0.3}Mn_{0.2}O₂, NaNi_{1/3}Ti_{1/3}Mn_{1/3}O₂, NaNi_{0.33}Ti_{0.33}Mn_{0.16}Mg_{0.17}O₂, Na_{2/3}Ni_{1/3}Ti_{1/6}Mn_{1/2}O₂, and Na_{2/3}Ni_{1/3}Mn_{2/3}O₂, sodium-containing transition metal oxide composites including a mixture of a plurality of transition metals in the sodium-containing transition metal oxide composites such as Co, Mn, and Ni, sodium-containing transition metal oxide composites in which a part of the transition metals in the sodium-containing transition metal oxide composites is substituted with a metal other than the transition metals, polyanion type compounds such as NaFePO₄, NaVPO₄F, Na₃V₂(PO₄)₃, and Na₂Fe₂(SO₄)₃, sodium salts of Prussian Blue analogues represented by a compositional formula NaₐM_{b}[Fe(CN)₆]_{c} (M represents Cr, Mn, Fe, Co, Ni, Cu, or Zn, 0 ≤ a ≤ 2, 0.5 ≤ b ≤ 1.5, and 0.5 ≤ c ≤ 1.5), oxides such as TiO₂, V₂O₅, and MoO₃, sulfides such as TiS₂ and FeS, conductive polymers such as polyacetylene, polyparaphenylene, polyaniline, and polypyrrole, activated carbon, radical-generating polymers, carbon materials, and the like may be used as a positive electrode material (positive electrode active material).

Acetylene black, Ketjen black, carbon fibers, or graphite as a conductive material and polytetrafluoroethylene, polyvinylidene fluoride, SBR resin, or the like as a binder may be added to the positive electrode and negative electrode materials, and an electrode sheet molded into a sheet shape may also be used.

As a separator for preventing contact between the positive electrode and the negative electrode, a nonwoven fabric or a porous sheet made of polypropylene, polyethylene, paper, glass fibers, or the like may be used.

An electrochemical device having a shape such as a coin shape, a cylindrical shape, a square shape, or an aluminum laminate sheet shape is assembled with the above elements.

### Examples

Hereinafter, the present disclosure will be described in more detail with Examples, but the present disclosure is not restricted by these descriptions.

### <Synthesis Example 1-1>

### Synthesis of Compound (1-1)

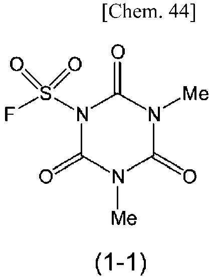

N,N-dimethylisocyanuric acid was synthesized referring to a document (European Journal of Organic Chemistry 2017, 4, 833).

To a 50-ml pear-shaped flask, 1.6 g of N,N-dimethylisocyanuric acid, 30 g of acetonitrile, and 0.10 g of LiH were charged, and then 1.2 g of sulfuryl fluoride was bubbled into the reaction solution at room temperature. After foaming ceased, the mixture was stirred at room temperature for 18 hours. The reaction solution was filtered, and the filtrate was concentrated to obtain 1.9 g (recovery rate: 79%) of Compound (1-1).
¹H NMR(CD₃CN) σ_{H} 3.25 ppm,
¹⁹F NMR (CD₃CN) σ_{F} 59.2 ppm.

### <Synthesis Example 1-2>

### Synthesis of Compound (1-2)

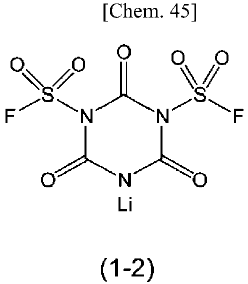

To a 50-ml pear-shaped flask, 1.3 g of isocyanuric acid, 30 g of acetonitrile, and 0.29 g of LiH were charged, and then 2.3 g of sulfuryl fluoride was bubbled into the reaction solution at room temperature. After foaming ceased, the mixture was stirred at room temperature for 16 hours. The reaction solution was filtered, and the filtrate was concentrated to obtain 1.8 g (recovery rate: 61%) of Compound (1-2).

¹⁹F NMR (CD₃CN) σ_{F} 46.6 ppm.

### <Synthesis Example 1-3>

### Synthesis of Compound (1-3)

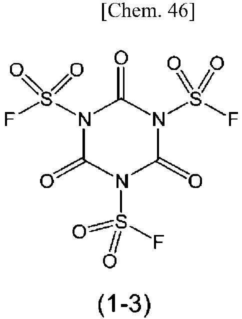

To a 50-ml pear-shaped flask, 1.3 g of fluorosulfonyl isocyanate, 30 g of toluene, and 0.08 g of 1-methylimidazole were charged, and then the mixture was stirred under the reflux condition at 120°C for 16 hours. The reaction solution was filtered, and the filtrate was concentrated to obtain 0.8 g (recovery rate: 61%) of Compound (1-3).

¹⁹F NMR (CD₃CN) σ_{F} 49.1 ppm.

### <Synthesis Example 1-4>

### Synthesis of Compound (1-4)

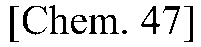

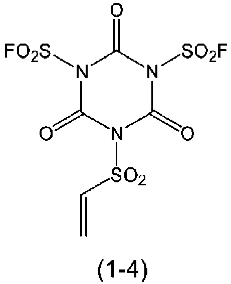

To a 50-ml pear-shaped flask, 1.5 g of the Compound (1-2), 30 g of acetonitrile, and 0.7 g of ethenesulfonyl chloride were charged, and the mixture was stirred at room temperature for 20 hours. The reaction solution was filtered, and the filtrate was concentrated to obtain 1.5 g (recovery rate: 78%) of Compound (1-4).
¹H NMR (CD₃CN) σ_{H} 5.93, 6.14, 6.98 ppm,
¹⁹F NMR (CD₃CN) σ_{F} 47.1 ppm.

### <Synthesis Example 1-5>

### Synthesis of Compound (1-5)

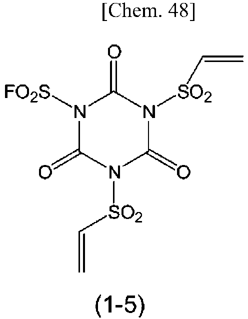

To a 50-ml pear-shaped flask, 1.8 g of the Compound (1-3) and 30 g of tetrahydrofuran were charged, and 10.0 g of 14% vinylmagnesium bromide/tetrahydrofuran was slowly added dropwise under ice cooling. The mixture was heated to room temperature and stirred for 15 hours. To the reaction solution, 100 g of an aqueous saturated ammonium chloride solution and 200 g of dichloromethane were added, and after mixing and separation, the organic layer was concentrated. The target product was separated and concentrated using silica gel column chromatography (developing solvent: dichloromethane) for the concentrated liquid to obtain 0.2 g (recovery rate: 10%) of Compound (1-5).
¹H NMR (CD₃CN) σ_{H} 5.83, 6.04, 6.88 ppm,
¹⁹F NMR (CD₃CN) σ_{F} 46.9 ppm.

### <Synthesis Example 2-1>

### Synthesis of Compound (2-1)

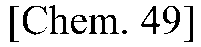

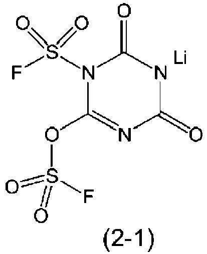

To a 50-ml pear-shaped flask, 1.6 g of Compound (2-2) described below, 30 g of acetonitrile, and 0.7 g of lithium iodide were charged, and the mixture was stirred at 40°C for five hours. The reaction solution was concentrated to obtain 1.4 g (recovery rate: 93%) of Compound (2-1).

¹⁹F NMR (CD₃CN) σ_{F} 46.5, 46.7 ppm.

### <Synthesis Example 2-2>

### Synthesis of Compound (2-2)

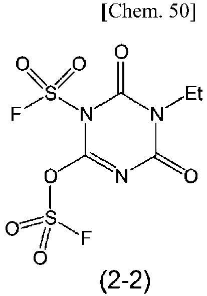

To a 50-ml pear-shaped flask, 2.7 g of fluorosulfonyl isocyanate, 0.7 g of ethyl isocyanate, and 0.08 g of 1-methylimidazole were charged, and then the mixture was stirred under the reflux condition at 80°C for 19 hours. The target product was separated and concentrated using silica gel column chromatography (developing solvent: dichloromethane/hexane) for the reaction solution to obtain 0.3 g (recovery rate: 19%) of Compound (2-2).
¹H NMR (CD₃CN) σ_{H} 1.23, 3.98 ppm,
¹⁹F NMR (CD₃CN) σ_{F} 59.7, 60.1 ppm.

The Compound (1-2Na) and the Compound (2-1Na) below were obtained by subjecting the Compound (1-2) and the Compound (2-1) to a cation exchange reaction, respectively.

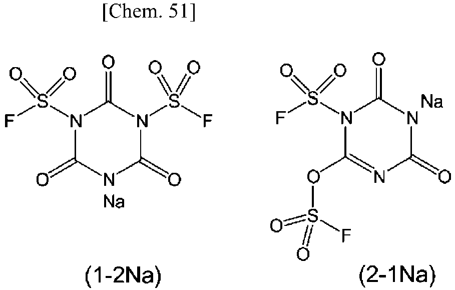

### [Preparation of Nonaqueous Electrolyte Solutions of Examples and Comparative Examples] <Electrolyte Solution for Lithium Ion Battery for Natural Graphite Negative Electrode>

### (Preparation of Nonaqueous Electrolyte Solutions Shown in Tables 1 to 8)

EC, PC, EMC, and DMC were mixed in a volume ratio of EC:PC:EMC:DMC = 25:5:45:25 in a glove box with a dew point of -60°C or lower.

Thereafter, LiPF₆ as a solute was dissolved in a concentration of 1.0 mol/L with respect to the total amount of the electrolyte solution, and at least one selected from the group consisting of the compound represented by the general formula (1) and the compound represented by the general formula (2) (component (I)) or the comparative compounds shown in Tables 1 to 8 below was dissolved in the amount shown in the tables below with respect to the total amount of the electrolyte solution. In Comparative Examples 1-1, 2-1, 3-1, 4-1, 5-1, 6-1, 7-1, and 8-1, neither the component (I) nor the comparative compound was added.

In addition, in the case of containing other additives, the compounds shown in Tables 1 to 8 below were similarly dissolved in the amount shown. The above preparation was performed at 25°C.

The Compound X was synthesized by the same method as that in JP7076527B. The Compound Y was synthesized by the same method as that in JP2019-50135A.

The structures of the Compound X and the Compound Y used in the Comparative Examples are shown below.

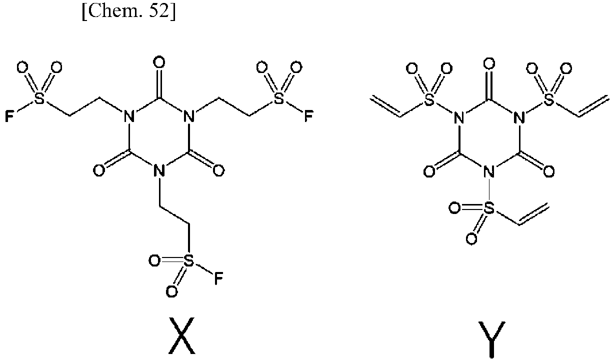

### <Electrolyte Solution for Lithium Ion Battery for Silicon-Containing Graphite Negative Electrode>

### (Preparation of Nonaqueous Electrolyte Solutions Shown in Tables 9 to 16)

EC, FEC, EMC, and DMC were mixed in a volume ratio of EC:FEC:EMC:DMC = 24:3:48:25 in a glove box with a dew point of -60°C or lower.

Thereafter, LiPF₆ as a solute was dissolved in a concentration of 1.0 mol/L with respect to the total amount of the electrolyte solution, and the component (I) or the comparative compound shown in Tables 9 to 16 below was dissolved in the amount shown in the tables below with respect to the total amount of the electrolyte solution. In Comparative Examples 9-1, 10-1, 11-1, 12-1, 13-1, 14-1, 15-1, and 16-1, neither the component (I) nor the comparative compound was added.

In addition, in the case of containing other additives, the compound shown in Tables 9 to 16 below was similarly dissolved in the amount shown. The above preparation was performed at 25°C.

### <Electrolyte Solution for Sodium Ion Battery>

### (Preparation of Nonaqueous Electrolyte Solutions Shown in Tables 17 to 23)

PC, EC, FEC, EMC, and DEC were mixed in a volume ratio of PC:EC:FEC:EMC:DEC = 20:8:2:48:22 in a glove box with a dew point of -60°C or lower.

Thereafter, NaPF₆ as a solute was dissolved in a concentration of 1.0 mol/L with respect to the total amount of the electrolyte solution, and the component (I) or the comparative compound shown in Tables 17 to 23 below was dissolved in the amount shown in the tables below with respect to the total amount of the electrolyte solution. In Comparative Examples 17-1, 18-1, 19-1, 20-1, 21-1, 22-1, and 23-1, neither the component (I) nor the comparative compound was added.

In addition, in the case of containing other additives, the compounds shown in Tables 17 to 23 below were similarly dissolved in the amount shown. The above preparation was performed at 25°C.

In Tables 1 to 23 below, VC means vinylene carbonate, BOB means lithium bis(oxalato)borate, DFBOP means lithium difluorobis(oxalato)phosphate, TFOP means lithium tetrafluorooxalatophosphate, FSI means lithium bis(fluorosulfonyl)imide, DFP means lithium difluorophosphate, FS means lithium fluorosulfonate, DTD means 1,3,2-dioxathiolane-2,2-dioxide, NaSO₃F means sodium fluorosulfonate, TFOP-Na means sodium tetrafluorooxalatophosphate, DFP-Na means sodium difluorophosphate, and DFOB-Na means sodium difluorooxalatoborate.

In Tables 1 to 23 below, the component (I) (or comparative compound) contents are the concentrations with respect to the total amounts of the nonaqueous electrolyte solutions. Moreover, the other additive contents are the concentrations with respect to the total amounts of the nonaqueous electrolyte solutions.

### [Production of Nonaqueous Electrolyte Solution Battery]

### (Production of Lithium Ion Battery Positive Electrode: NCM622 Positive Electrode)

To 90% by mass of LiNi_{0.6}Co_{0.2}Mn_{0.2}O₂ powder, 5% by mass of polyvinylidene fluoride (hereinafter also referred to as PVDF) as a binder and 5% by mass of acetylene black as a conductive material were mixed, and N-methyl-2-pyrrolidone was further added to produce a positive electrode mixture paste. The paste was applied onto both sides of an aluminum foil (A1085), dried, pressed and then punched into 4 cm × 5 cm to obtain an NCM622 positive electrode for testing.

### (Production of Lithium Ion Battery Positive Electrode: NCM811 Positive Electrode)

To 92.0% by mass of LiNi_{0.8}Co_{0.1}Mn_{0.1}O₂ powder, 3.5% by mass of PVDF as a binder and 4.5% by mass of acetylene black as a conductive material were mixed, and N-methyl-2-pyrrolidone was further added to produce a positive electrode mixture paste. The paste was applied onto both sides of an aluminum foil (A1085), dried, pressed and then punched into 4 cm × 5 cm to obtain an NCM811 positive electrode for testing.

### (Production of Sodium Ion Battery Positive Electrode: NaNi_{0.5}Ti_{0.3}Mn_{0.2}O₂ Positive Electrode)

By mixing 90% by mass of NaNi_{0.3}Ti_{0.3}Mn_{0.2}O₂ as a positive electrode active material, 5% by mass of acetylene black as a conductive agent, and 5% by mass of PVDF as a binder and further adding N-methyl-2-pyrrolidone as a solvent in such a manner that the content thereof became 50% by mass with respect to the total mass of the positive electrode active material, the conductive agent, and the binder, a slurry solution was prepared. The slurry solution was applied onto an aluminum foil serving as a positive electrode current collector and dried at 150°C for 12 hours to obtain an NaNi_{0.3}Ti_{0.3}Mn_{0.2}O₂ positive electrode for testing in which a positive electrode active material layer was formed on the current collector.

### (Production of Natural Graphite Negative Electrode)

By mixing 92% by mass of natural graphite powder, 3% by mass of a conductive material (HS-100), 2% by mass of carbon nanofiber (VGCF), 2% by mass of styrene-butadiene rubber, 1% by mass of sodium carboxymethyl cellulose, and water, a slurry solution was prepared. The slurry solution was applied onto a copper foil serving as a negative electrode current collector and dried at 100°C for 12 hours to obtain a natural graphite negative electrode for testing in which a negative electrode active material layer was formed on the current collector.

### (Production of Silicon-Containing Graphite Negative Electrode)

To 85% by mass of artificial graphite powder, 7% by mass of nanosilicon, 3% by mass of a conductive material (HS-100), 2% by mass of carbon nanofiber (VGCF), 2% by mass of styrene-butadiene rubber, 1% by mass of sodium carboxymethyl cellulose, and water were mixed to prepare a slurry solution. The slurry solution was applied onto a copper foil serving as a negative electrode current collector and dried at 100°C for 12 hours to obtain a silicon-containing graphite negative electrode for testing in which a negative electrode active material layer was formed on the current collector.

### (Production of Hard Carbon Negative Electrode)

By mixing 90% by mass of hard carbon powder (manufactured by KUREHA CORPORATION, Carbotron P) and 10% by mass of PVDF as a binder and further adding N-methylpyrrolidone as a solvent in such a manner that the content thereof became 50% by mass with respect to the total mass of the negative electrode active material and the binder, a slurry solution was prepared. The slurry solution was applied onto an aluminum foil serving as a negative electrode current collector and dried at 150°C for 12 hours to obtain a hard carbon negative electrode for testing in which a negative electrode active material layer was formed on the current collector.

### (Production of Nonaqueous Electrolyte Solution Battery)

Under an argon atmosphere at a dew point of -50°C or lower, a terminal was welded to the above NCM622 positive electrode, and both sides of the welded product were then sandwiched between two polyethylene separators (5 cm × 6 cm). The outside of the sandwiched product was sandwiched between two natural graphite negative electrodes to which a terminal had been welded in advance, in such a manner that the surface of the negative electrode active material faced the surface of the positive electrode active material. The resultant product was put in an aluminum laminated bag having an opening on one side, and after the nonaqueous electrolyte solution was vacuum-injected into the bag, the opening was sealed with heat. In this manner, the aluminum laminated nonaqueous electrolyte solution batteries of Examples and Comparative Examples were produced. The nonaqueous electrolyte solutions shown in Tables 1 to 8 were used.

The nonaqueous electrolyte solution batteries according to Tables 9 to 16 were produced by the same procedure as described above except for changing the positive electrode to the NCM811 positive electrode described above, changing the negative electrode to the silicon-containing graphite negative electrode described above and using those shown in Tables 9 to 16 as the nonaqueous electrolyte solutions.

In addition, the nonaqueous electrolyte solution batteries according to Tables 17 to 23 were produced by the same procedure as described above except for changing the positive electrode to the sodium ion battery positive electrode described above, changing the negative electrode to the hard carbon negative electrode described above and using those shown in Tables 17 to 23 as the nonaqueous electrolyte solutions.

### [Evaluation]

### -Lithium Ion Battery: Initial Charge and Discharge-

The produced nonaqueous electrolyte solution battery was put in a 25°C constant temperature bath and, in this state, connected to a charge and discharge device. Charge was performed at 3 mA to 4.3 V. After 4.3 V was maintained for one hour, discharge was performed at 6 mA to 2.5 V. This was defined as one charge and discharge cycle, and three cycles of charge and discharge in total were performed to stabilize the battery.

### -Sodium Ion Battery: Initial Charge and Discharge-

The produced nonaqueous electrolyte solution battery was put in a 25°C constant temperature bath and, in this state, connected to a charge and discharge device. Charge was performed at 3 mA to 4.1 V. After 4.1 V was maintained for one hour, discharge was performed at 6 mA to 1.5 V. This was defined as one charge and discharge cycle, and three cycles of charge and discharge in total were performed to stabilize the battery.

### [Lithium Ion Battery: Initial Resistance Measurement]

The test cell was charged to 4.3 V at 25°C and 6 mA, and then the resistance value was measured by impedance measurement in a -10°C environment.

### [Sodium Ion Battery: Initial Resistance Measurement]

Except that the charge voltage was changed to 4.1 V, the evaluation was performed in the same manner as that for the lithium ion battery.

### [Lithium Ion Battery: High-Temperature Cycle Characteristic Evaluation]

The test cell was subjected to a charge and discharge test at an ambient temperature of 50°C to evaluate the cycle characteristic. A charge and discharge cycle was repeated at a current value of 30 mA using a constant current constant voltage method, with an upper limit charge voltage of 4.3 V and a lower limit discharge voltage of 2.5 V. Further, the degree of deterioration of the cell was evaluated with the discharge capacity retention rate at the 500th cycle in the charge and discharge test at an ambient temperature of 50°C. The "discharge capacity retention rate after high-temperature cycles" expressed as the discharge capacity retention rate at the 500th cycle was calculated using the following equation. The discharge capacity at the first cycle in the charge and discharge test at the ambient temperature of 50°C was defined as the initial discharge capacity. Discharge capacity retention rate (%) after high-temperature cycles = (discharge capacity at 500th cycle/initial discharge capacity) × 100

### [Sodium Ion Battery: Evaluation of High-Temperature Cycle Characteristic]

Except that the upper limit charge voltage was changed to 4.1 V and that the lower limit discharge voltage was changed to 1.5 V, evaluation was performed in the same manner as that for the lithium ion battery.

In each of Tables 1 to 23, the initial resistance value and the discharge capacity retention rate after the high-temperature cycles of Comparative Examples using the comparative nonaqueous electrolyte solutions to which neither the component (I) nor the comparative compounds was added (Comparative Example 1-1 in Table 1, Comparative Example 2-1 in Table 2, Comparative Example 3-1 in Table 3, Comparative Example 4-1 in Table 4, Comparative Example 5-1 in Table 5, Comparative Example 6-1 in Table 6, Comparative Example 7-1 in Table 7, Comparative Example 8-1 in Table 8, Comparative Example 9-1 in Table 9, Comparative Example 10-1 in Table 10, Comparative Example 11-1 in Table 11, Comparative Example 12-1 in Table 12, Comparative Example 13-1 in Table 13, Comparative Example 14-1 in Table 14, Comparative Example 15-1 in Table 15, Comparative Example 16-1 in Table 16, Comparative Example 17-1 in Table 17, Comparative Example 18-1 in Table 18, Comparative Example 19-1 in Table 19, Comparative Example 20-1 in Table 20, Comparative Example 21-1 in Table 21, Comparative Example 22-1 in Table 22, and Comparative Example 23-1 in Table 23) were defined as 100, and the relative values of the evaluation results of Examples and Comparative Examples were shown.

### [Table 1]

**Table 1**

| | Nonaqueous Electrolyte Solution | Component (I) or Comparative Compound | | Other Additive | | Initial Resistance (relative value) | Discharge Capacity Retention Rate (relative value) |
|---|---|---|---|---|---|---|---|
| | | Type | Addition Amount [% by mass] | Type | Addition Amount [% by mass] | | |
| Example 1-1 | Nonaqueous electrolyte solution 1-1 | (1-1) | 0.05 | - | - | 95.7 | 100.2 |
| Example 1-2 | Nonaqueous electrolyte solution 1-2 | (1-1) | 0.1 | - | - | 94.6 | 101.1 |
| Example 1-3 | Nonaqueous electrolyte solution 1-3 | (1-1) | 0.5 | - | - | 90.1 | 104.7 |
| Example 1-4 | Nonaqueous electrolyte solution 1-4 | (1-1) | 1.0 | - | - | 84.2 | 106.7 |
| Example 1-5 | Nonaqueous electrolyte solution 1-5 | (1-1) | 2.0 | - | - | 91.8 | 107.1 |
| Example 1-6 | Nonaqueous electrolyte solution 1-6 | (1-1) | 3.0 | - | - | 96.0 | 103.1 |
| Example 1-7 | Nonaqueous electrolyte solution 1-7 | (1-2) | 0.5 | - | - | 85.2 | 105.8 |
| Example 1-8 | Nonaqueous electrolyte solution 1-8 | (1-2) | 1.0 | - | - | 82.9 | 107.0 |
| Example 1-9 | Nonaqueous electrolyte solution 1-9 | (1-4) | 0.5 | - | - | 96.8 | 104.0 |
| Example 1-10 | Nonaqueous electrolyte solution 1-10 | (1-4) | 1.0 | - | - | 96.5 | 105.9 |
| Example 1-11 | Nonaqueous electrolyte solution 1-11 | (1-5) | 0.5 | - | - | 97.0 | 102.8 |
| Example 1-12 | Nonaqueous electrolyte solution 1-12 | (1-5) | 1.0 | - | - | 99.4 | 104.7 |
| Example 1-13 | Nonaqueous electrolyte solution 1-13 | (2-1) | 0.5 | - | - | 84.3 | 105.9 |
| Example 1-14 | Nonaqueous electrolyte solution 1-14 | (2-1) | 1.0 | - | - | 82.5 | 107.1 |
| Example 1-15 | Nonaqueous electrolyte solution 1-15 | (2-2) | 0.5 | - | - | 85.5 | 104.8 |
| Example 1-16 | Nonaqueous electrolyte solution 1-16 | (2-2) | 1.0 | - | - | 83.7 | 106.0 |
| Comparative Example 1-1 | Comparative nonaqueous electrolyte solution 1-1 | - | - | - | - | 100.0 | 100.0 |
| Comparative Example 1-2 | Comparative nonaqueous electrolyte solution 1-2 | X | 1.0 | - | - | 110.2 | 99.3 |
| Comparative Example 1-3 | Comparative nonaqueous electrolyte solution 1-3 | Y | 1.0 | - | - | 136.6 | 102.5 |

### [Table 2]

**Table 2**

| | Nonaqueous Electrolyte Solution | Component (I) or Comparative Compound | | Other Additive | | Initial Resistance (relative value) | Discharge Capacity Retention Rate (relative value) |
|---|---|---|---|---|---|---|---|
| | | Type | Addition Amount [% by mass] | Type | Addition Amount [% by mass] | | |
| Example 2-1 | Nonaqueous electrolyte solution 2-1 | (1-1) | 1.0 | VC | 1.0 | 85.9 | 104.4 |
| Example 2-2 | Nonaqueous electrolyte solution 2-2 | (1-2) | 1.0 | VC | 1.0 | 81.5 | 105.3 |
| Example 2-3 | Nonaqueous electrolyte solution 2-3 | (1-4) | 1.0 | VC | 1.0 | 94.2 | 104.0 |
| Comparative Example 2-1 | Comparative nonaqueous electrolyte solution 2-1 | - | - | VC | 1.0 | 100.0 | 100.0 |
| Comparative Example 2-2 | Comparative nonaqueous electrolyte solution 2-2 | X | 1.0 | VC | 1.0 | 106.9 | 101.2 |
| Comparative Example 2-3 | Comparative nonaqueous electrolyte solution 2-3 | Y | 1.0 | VC | 1.0 | 127.3 | 103.4 |

### [Table 3]

**Table 3**

| | Nonaqueous Electrolyte Solution | Component (I) or Comparative Compound | | Other Additive | | Initial Resistance (relative value) | Discharge Capacity Retention Rate (relative value) |
|---|---|---|---|---|---|---|---|
| | | Type | Addition Amount [% by mass] | Type | Addition Amount [% by mass] | | |
| Example 3-1 | Nonaqueous electrolyte solution 3-1 | (1-1) | 1.0 | BOB | 1.0 | 84.8 | 103.4 |
| Example 3-2 | Nonaqueous electrolyte solution 3-2 | (1-2) | 1.0 | BOB | 1.0 | 80.4 | 104.7 |
| Example 3-3 | Nonaqueous electrolyte solution 3-3 | (1-4) | 1.0 | BOB | 1.0 | 93.5 | 103.1 |
| Comparative Example 3-1 | Comparative nonaqueous electrolyte solution 3-1 | - | - | BOB | 1.0 | 100.0 | 100.0 |
| Comparative Example 3-2 | Comparative nonaqueous electrolyte solution 3-2 | X | 1.0 | BOB | 1.0 | 105.6 | 100.6 |
| Comparative Example 3-3 | Comparative nonaqueous electrolyte solution 3-3 | Y | 1.0 | BOB | 1.0 | 117.8 | 102.4 |

### [Table 4]

**Table 4**

| | Nonaqueous Electrolyte Solution | Component (I) or Comparative Compound | | Other Additive | | Initial Resistance (relative value) | Discharge Capacity Retention Rate (relative value) |
|---|---|---|---|---|---|---|---|
| | | Type | Addition Amount [% by mass] | Type | Addition Amount [% by mass] | | |
| Example 4-1 | Nonaqueous electrolyte solution 4-1 | (1-1) | 1.0 | DFBOP | 1.0 | 82.0 | 104.4 |
| Example 4-2 | Nonaqueous electrolyte solution 4-2 | (1-2) | 1.0 | DFBOP | 1.0 | 78.9 | 105.7 |
| Example 4-3 | Nonaqueous electrolyte solution 4-3 | (1-4) | 1.0 | DFBOP | 1.0 | 92.1 | 103.7 |
| Comparative Example 4-1 | Comparative nonaqueous electrolyte solution 4-1 | - | - | DFBOP | 1.0 | 100.0 | 100.0 |
| Comparative Example 4-2 | Comparative nonaqueous electrolyte solution 4-2 | X | 1.0 | DFBOP | 1.0 | 101.7 | 100.8 |
| Comparative Example 4-3 | Comparative nonaqueous electrolyte solution 4-3 | Y | 1.0 | DFBOP | 1.0 | 119.3 | 102.5 |

### [Table 5]

**Table 5**

| | Nonaqueous Electrolyte Solution | Component (I) or Comparative Compound | | Other Additive | | Initial Resistance (relative value) | Discharge Capacity Retention Rate (relative value) |
|---|---|---|---|---|---|---|---|
| | | Type | Addition Amount [% by mass] | Type | Addition Amount [% by mass] | | |
| Example 5-1 | Nonaqueous electrolyte solution 5-1 | (1-1) | 1.0 | TFOP | 1.0 | 83.1 | 104.4 |
| Example 5-2 | Nonaqueous electrolyte solution 5-2 | (1-2) | 1.0 | TFOP | 1.0 | 78.7 | 106.9 |
| Example 5-3 | Nonaqueous electrolyte solution 5-3 | (1-4) | 1.0 | TFOP | 1.0 | 89.5 | 104.0 |
| Comparative Example 5-1 | Comparative nonaqueous electrolyte solution 5-1 | - | - | TFOP | 1.0 | 100.0 | 100.0 |
| Comparative Example 5-2 | Comparative nonaqueous electrolyte solution 5-2 | X | 1.0 | TFOP | 1.0 | 105.3 | 101.4 |
| Comparative Example 5-3 | Comparative nonaqueous electrolyte solution 5-3 | Y | 1.0 | TFOP | 1.0 | 128.3 | 102.3 |

### [Table 6]

**Table 6**

| | Nonaqueous Electrolyte Solution | Component (I) or Comparative Compound | | Other Additive | | Initial Resistance (relative value) | Discharge Capacity Retention Rate (relative value) |
|---|---|---|---|---|---|---|---|
| | | Type | Addition Amount [% by mass] | Type | Addition Amount [% by mass] | | |
| Example 6-1 | Nonaqueous electrolyte solution 6-1 | (1-1) | 1.0 | FSI | 1.0 | 82.2 | 106.1 |
| Example 6-2 | Nonaqueous electrolyte solution 6-2 | (1-2) | 1.0 | FSI | 1.0 | 79.9 | 107.7 |
| Example 6-3 | Nonaqueous electrolyte solution 6-3 | (1-4) | 1.0 | FSI | 1.0 | 88.8 | 104.1 |
| Comparative Example 6-1 | Comparative nonaqueous electrolyte solution 6-1 | - | - | FSI | 1.0 | 100.0 | 100.0 |
| Comparative Example 6-2 | Comparative nonaqueous electrolyte solution 6-2 | X | 1.0 | FSI | 1.0 | 103.5 | 101.8 |
| Comparative Example 6-3 | Comparative nonaqueous electrolyte solution 6-3 | Y | 1.0 | FSI | 1.0 | 119.5 | 102.5 |

### [Table 7]

**Table 7**

| | Nonaqueous Electrolyte Solution | Component (I) or Comparative Compound | | Other Additive | | Initial Resistance (relative value) | Discharge Capacity Retention Rate (relative value) |
|---|---|---|---|---|---|---|---|
| | | Type | Addition Amount [% by mass] | Type | Addition Amount [% by mass] | | |
| Example 7-1 | Nonaqueous electrolyte solution 7-1 | (1-1) | 1.0 | DFP | 1.0 | 83.9 | 103.9 |
| Example 7-2 | Nonaqueous electrolyte solution 7-2 | (1-2) | 1.0 | DFP | 1.0 | 81.8 | 106.1 |
| Example 7-3 | Nonaqueous electrolyte solution 7-3 | (1-4) | 1.0 | DFP | 1.0 | 93.3 | 103.4 |
| Comparative Example 7-1 | Comparative nonaqueous electrolyte solution 7-1 | - | - | DFP | 1.0 | 100.0 | 100.0 |
| Comparative Example 7-2 | Comparative nonaqueous electrolyte solution 7-2 | X | 1.0 | DFP | 1.0 | 104.3 | 101.4 |
| Comparative Example 7-3 | Comparative nonaqueous electrolyte solution 7-3 | Y | 1.0 | DFP | 1.0 | 121.5 | 102.1 |

### [Table 8]

**Table 8**

| | Nonaqueous Electrolyte Solution | Component (I) or Comparative Compound | | Other Additive | | Initial Resistance (relative value) | Discharge Capacity Retention Rate (relative value) |
|---|---|---|---|---|---|---|---|
| | | Type | Addition Amount [% by mass] | Type | Addition Amount [% by mass] | | |
| Example 8-1 | Nonaqueous electrolyte solution 8-1 | (1-1) | 1.0 | FS | 1.0 | 87.9 | 102.7 |
| Example 8-2 | Nonaqueous electrolyte solution 8-2 | (1-2) | 1.0 | FS | 1.0 | 85.7 | 104.1 |
| Example 8-3 | Nonaqueous electrolyte solution 8-3 | (1-4) | 1.0 | FS | 1.0 | 95.1 | 102.4 |
| Comparative Example 8-1 | Comparative nonaqueous electrolyte solution 8-1 | - | - | FS | 1.0 | 100.0 | 100.0 |
| Comparative Example 8-2 | Comparative nonaqueous electrolyte solution 8-2 | X | 1.0 | FS | 1.0 | 104.9 | 101.4 |
| Comparative Example 8-3 | Comparative nonaqueous electrolyte solution 8-3 | Y | 1.0 | FS | 1.0 | 116.6 | 101.9 |

### [Table 9]

**Table 9**

| | Nonaqueous Electrolyte Solution | Component (I) or Comparative Compound | | Other Additive | | Initial Resistance (relative value) | Discharge Capacity Retention Rate (relative value) |
|---|---|---|---|---|---|---|---|
| | | Type | Addition Amount [% by mass] | Type | Addition Amount [% by mass] | | |
| Example 9-1 | Nonaqueous electrolyte solution 9-1 | (1-1) | 0.05 | - | - | 94.3 | 101.5 |
| Example 9-2 | Nonaqueous electrolyte solution 9-2 | (1-1) | 0.1 | - | - | 92.8 | 102.8 |
| Example 9-3 | Nonaqueous electrolyte solution 9-3 | (1-1) | 0.5 | - | - | 83.5 | 105.8 |
| Example 9-4 | Nonaqueous electrolyte solution 9-4 | (1-1) | 1.0 | - | - | 75.2 | 107.2 |
| Example 9-5 | Nonaqueous electrolyte solution 9-5 | (1-1) | 2.0 | - | - | 81.1 | 107.3 |
| Example 9-6 | Nonaqueous electrolyte solution 9-6 | (1-1) | 3.0 | - | - | 89.6 | 105.1 |
| Example 9-7 | Nonaqueous electrolyte solution 9-7 | (1-2) | 0.5 | - | - | 78.1 | 109.4 |
| Example 9-8 | Nonaqueous electrolyte solution 9-8 | (1-2) | 1.0 | - | - | 70.3 | 112.5 |
| Example 9-9 | Nonaqueous electrolyte solution 9-9 | (1-4) | 0.5 | - | - | 88.9 | 104.0 |
| Example 9-10 | Nonaqueous electrolyte solution 9-10 | (1-4) | 1.0 | - | - | 87.1 | 106.5 |
| Example 9-11 | Nonaqueous electrolyte solution 9-11 | (1-5) | 0.5 | - | - | 98.7 | 102.0 |
| Example 9-12 | Nonaqueous electrolyte solution 9-12 | (1-5) | 1.0 | - | - | 99.8 | 104.8 |
| Example 9-13 | Nonaqueous electrolyte solution 9-13 | (2-1) | 0.5 | - | - | 76.7 | 110.9 |
| Example 9-14 | Nonaqueous electrolyte solution 9-14 | (2-1) | 1.0 | - | - | 70.0 | 115.3 |
| Example 9-15 | Nonaqueous electrolyte solution 9-15 | (2-2) | 0.5 | - | - | 79.9 | 107.0 |
| Example 9-16 | Nonaqueous electrolyte solution 9-16 | (2-2) | 1.0 | - | - | 73.1 | 110.1 |
| Comparative Example 9-1 | Comparative nonaqueous electrolyte solution 9-1 | - | - | - | - | 100.0 | 100.0 |
| Comparative Example 9-2 | Comparative nonaqueous electrolyte solution 9-2 | X | 1.0 | - | - | 115.2 | 98.2 |
| Comparative Example 9-3 | Comparative nonaqueous electrolyte solution 9-3 | Y | 1.0 | - | - | 149.8 | 101.4 |

### [Table 10]

**Table 10**

| | Nonaqueous Electrolyte Solution | Component (I) or Comparative Compound | | Other Additive | | Initial Resistance (relative value) | Discharge Capacity Retention Rate (relative value) |
|---|---|---|---|---|---|---|---|
| | | Type | Addition Amount [% by mass] | Type | Addition Amount [% by mass] | | |
| Example 10-1 | Nonaqueous electrolyte solution 10-1 | (1-1) | 1.0 | VC | 1.0 | 72.5 | 106.5 |
| Example 10-2 | Nonaqueous electrolyte solution 10-2 | (1-2) | 1.0 | VC | 1.0 | 70.1 | 111.7 |
| Example 10-3 | Nonaqueous electrolyte solution 10-3 | (1-4) | 1.0 | VC | 1.0 | 85.8 | 106.3 |
| Comparative Example 10-1 | Comparative nonaqueous electrolyte solution 10-1 | - | - | VC | 1.0 | 100.0 | 100.0 |
| Comparative Example 10-2 | Comparative nonaqueous electrolyte solution 10-2 | X | 1.0 | VC | 1.0 | 118.1 | 100.7 |
| Comparative Example 10-3 | Comparative nonaqueous electrolyte solution 10-3 | Y | 1.0 | VC | 1.0 | 138.8 | 101.9 |

### [Table 11]

**Table 11**

| | Nonaqueous Electrolyte Solution | Component (I) or Comparative Compound | | Other Additive | | Initial Resistance (relative value) | Discharge Capacity Retention Rate (relative value) |
|---|---|---|---|---|---|---|---|
| | | Type | Addition Amount [% by mass] | Type | Addition Amount [% by mass] | | |
| Example 11-1 | Nonaqueous electrolyte solution 11-1 | (1-1) | 1.0 | BOB | 1.0 | 81.3 | 106.9 |
| Example 11-2 | Nonaqueous electrolyte solution 11-2 | (1-2) | 1.0 | BOB | 1.0 | 74.7 | 109.5 |
| Example 11-3 | Nonaqueous electrolyte solution 11-3 | (1-4) | 1.0 | BOB | 1.0 | 85.9 | 105.4 |
| Comparative Example 11-1 | Comparative nonaqueous electrolyte solution 11-1 | - | - | BOB | 1.0 | 100.0 | 100.0 |
| Comparative Example 11-2 | Comparative nonaqueous electrolyte solution 11-2 | X | 1.0 | BOB | 1.0 | 115.9 | 100.4 |
| Comparative Example 11-3 | Comparative nonaqueous electrolyte solution 11-3 | Y | 1.0 | BOB | 1.0 | 129.3 | 101.7 |

### [Table 12]

**Table 12**

| | Nonaqueous Electrolyte Solution | Component (I) or Comparative Compound | | Other Additive | | Initial Resistance (relative value) | Discharge Capacity Retention Rate (relative value) |
|---|---|---|---|---|---|---|---|
| | | Type | Addition Amount [% by mass] | Type | Addition Amount [% by mass] | | |
| Example 12-1 | Nonaqueous electrolyte solution 12-1 | (1-1) | 1.0 | DFBOP | 1.0 | 84.4 | 106.9 |
| Example 12-2 | Nonaqueous electrolyte solution 12-2 | (1-2) | 1.0 | DFBOP | 1.0 | 78.2 | 109.5 |
| Example 12-3 | Nonaqueous electrolyte solution 12-3 | (1-4) | 1.0 | DFBOP | 1.0 | 88.5 | 105.4 |
| Comparative Example 12-1 | Comparative nonaqueous electrolyte solution 12-1 | - | - | DFBOP | 1.0 | 100.0 | 100.0 |
| Comparative Example 12-2 | Comparative nonaqueous electrolyte solution 12-2 | X | 1.0 | DFBOP | 1.0 | 111.3 | 101.1 |
| Comparative Example 12-3 | Comparative nonaqueous electrolyte solution 12-3 | Y | 1.0 | DFBOP | 1.0 | 122.4 | 101.9 |

### [Table 13]

**Table 13**

| | Nonaqueous Electrolyte Solution | Component (I) or Comparative Compound | | Other Additive | | Initial Resistance (relative value) | Discharge Capacity Retention Rate (relative value) |
|---|---|---|---|---|---|---|---|
| | | Type | Addition Amount [% by mass] | Type | Addition Amount [% by mass] | | |
| Example 13-1 | Nonaqueous electrolyte solution 13-1 | (1-1) | 1.0 | TFOP | 1.0 | 79.7 | 105.9 |
| Example 13-2 | Nonaqueous electrolyte solution 13-2 | (1-2) | 1.0 | TFOP | 1.0 | 75.1 | 112.2 |
| Example 13-3 | Nonaqueous electrolyte solution 13-3 | (1-4) | 1.0 | TFOP | 1.0 | 87.4 | 105.2 |
| Comparative Example 13-1 | Comparative nonaqueous electrolyte solution 13-1 | - | - | TFOP | 1.0 | 100.0 | 100.0 |
| Comparative Example 13-2 | Comparative nonaqueous electrolyte solution 13-2 | X | 1.0 | TFOP | 1.0 | 114.3 | 100.8 |
| Comparative Example 13-3 | Comparative nonaqueous electrolyte solution 13-3 | Y | 1.0 | TFOP | 1.0 | 133.4 | 102.1 |

### [Table 14]

**Table 14**

| | Nonaqueous Electrolyte Solution | Component (I) or Comparative Compound | | Other Additive | | Initial Resistance (relative value) | Discharge Capacity Retention Rate (relative value) |
|---|---|---|---|---|---|---|---|
| | | Type | Addition Amount [% by mass] | Type | Addition Amount [% by mass] | | |
| Example 14-1 | Nonaqueous electrolyte solution 14-1 | (1-1) | 1.0 | FSI | 1.0 | 81.7 | 107.8 |
| Example 14-2 | Nonaqueous electrolyte solution 14-2 | (1-2) | 1.0 | FSI | 1.0 | 76.9 | 111.8 |
| Example 14-3 | Nonaqueous electrolyte solution 14-3 | (1-4) | 1.0 | FSI | 1.0 | 88.5 | 106.4 |
| Comparative Example 14-1 | Comparative nonaqueous electrolyte solution 14-1 | - | - | FSI | 1.0 | 100.0 | 100.0 |
| Comparative Example 14-2 | Comparative nonaqueous electrolyte solution 14-2 | X | 1.0 | FSI | 1.0 | 112.4 | 100.1 |
| Comparative Example 14-3 | Comparative nonaqueous electrolyte solution 14-3 | Y | 1.0 | FSI | 1.0 | 128.5 | 101.4 |

### [Table 15]

**Table 15**

| | Nonaqueous Electrolyte Solution | Component (I) or Comparative Compound | | Other Additive | | Initial Resistance (relative value) | Discharge Capacity Retention Rate (relative value) |
|---|---|---|---|---|---|---|---|
| | | Type | Addition Amount [% by mass] | Type | Addition Amount [% by mass] | | |
| Example 15-1 | Nonaqueous electrolyte solution 15-1 | (1-1) | 1.0 | DFP | 1.0 | 80.3 | 108.3 |
| Example 15-2 | Nonaqueous electrolyte solution 15-2 | (1-2) | 1.0 | DFP | 1.0 | 77.2 | 111.8 |
| Example 15-3 | Nonaqueous electrolyte solution 15-3 | (1-4) | 1.0 | DFP | 1.0 | 89.3 | 107.1 |
| Comparative Example 15-1 | Comparative nonaqueous electrolyte solution 15-1 | - | - | DFP | 1.0 | 100.0 | 100.0 |
| Comparative Example 15-2 | Comparative nonaqueous electrolyte solution 15-2 | X | 1.0 | DFP | 1.0 | 114.1 | 100.7 |
| Comparative Example 15-3 | Comparative nonaqueous electrolyte solution 15-3 | Y | 1.0 | DFP | 1.0 | 118.4 | 102.1 |

### [Table 16]

**Table 16**

| | Nonaqueous Electrolyte Solution | Component (I) or Comparative Compound | | Other Additive | | Initial Resistance (relative value) | Discharge Capacity Retention Rate (relative value) |
|---|---|---|---|---|---|---|---|
| | | Type | Addition Amount [% by mass] | Type | Addition Amount [% by mass] | | |
| Example 16-1 | Nonaqueous electrolyte solution 16-1 | (1-1) | 1.0 | FS | 1.0 | 84.4 | 107.4 |
| Example 16-2 | Nonaqueous electrolyte solution 16-2 | (1-2) | 1.0 | FS | 1.0 | 79.1 | 110.2 |
| Example 16-3 | Nonaqueous electrolyte solution 16-3 | (1-4) | 1.0 | FS | 1.0 | 90.5 | 106.1 |
| Comparative Example 16-1 | Comparative nonaqueous electrolyte solution 16-1 | - | - | FS | 1.0 | 100.0 | 100.0 |
| Comparative Example 16-2 | Comparative nonaqueous electrolyte solution 16-2 | X | 1.0 | FS | 1.0 | 114.1 | 100.3 |
| Comparative Example 16-3 | Comparative nonaqueous electrolyte solution 16-3 | Y | 1.0 | FS | 1.0 | 121.6 | 101.4 |

### [Table 17]

**Table 17**

| | Nonaqueous Electrolyte Solution | Component (I) or Comparative Compound | | Other Additive | | Initial Resistance (relative value) | Discharge Capacity Retention Rate (relative value) |
|---|---|---|---|---|---|---|---|
| | | Type | Addition Amount [% by mass] | Type | Addition Amount [% by mass] | | |
| Example 17-1 | Nonaqueous electrolyte solution 17-1 | (1-1) | 0.5 | - | - | 73.3 | 108.3 |
| Example 17-2 | Nonaqueous electrolyte solution 17-2 | (1-1) | 1.0 | - | - | 72.4 | 109.8 |
| Example 17-3 | Nonaqueous electrolyte solution 17-3 | (1-2Na) | 0.5 | - | - | 66.8 | 112.5 |
| Example 17-4 | Nonaqueous electrolyte solution 17-4 | (1-2Na) | 1.0 | - | - | 63.0 | 118.7 |
| Example 17-5 | Nonaqueous electrolyte solution 17-5 | (1-4) | 0.5 | - | - | 79.1 | 107.2 |
| Example 17-6 | Nonaqueous electrolyte solution 17-6 | (1-4) | 1.0 | - | - | 80.7 | 108.1 |
| Example 17-7 | Nonaqueous electrolyte solution 17-7 | (1-5) | 0.5 | - | - | 89.9 | 104.7 |
| Example 17-8 | Nonaqueous electrolyte solution 17-8 | (1-5) | 1.0 | - | - | 92.1 | 106.1 |
| Example 17-9 | Nonaqueous electrolyte solution 17-9 | (2-1Na) | 0.5 | - | - | 68.9 | 111.7 |
| Example 17-10 | Nonaqueous electrolyte solution 17-10 | (2-1Na) | 1.0 | - | - | 65.5 | 118.2 |
| Example 17-11 | Nonaqueous electrolyte solution 17-11 | (2-2) | 0.5 | - | - | 78.0 | 106.3 |
| Example 17-12 | Nonaqueous electrolyte solution 17-12 | (2-2) | 1.0 | - | - | 79.4 | 107.7 |
| Comparative Example 17-1 | Comparative nonaqueous electrolyte solution 17-1 | - | - | - | - | 100.0 | 100.0 |
| Comparative Example 17-2 | Comparative nonaqueous electrolyte solution 17-2 | X | 1.0 | - | - | 112.7 | 102.1 |
| Comparative Example 17-3 | Comparative nonaqueous electrolyte solution 17-3 | Y | 1.0 | - | - | 161.3 | 102.4 |

### [Table 18]

**Table 18**

| | Nonaqueous Electrolyte Solution | Component (I) or Comparative Compound | | Other Additive | | Initial Resistance (relative value) | Discharge Capacity Retention Rate (relative value) |
|---|---|---|---|---|---|---|---|
| | | Type | Addition Amount [% by mass] | Type | Addition Amount [% by mass] | | |
| Example 18-1 | Nonaqueous electrolyte solution 18-1 | (1-1) | 1.0 | VC | 1.0 | 77.9 | 109.3 |
| Example 18-2 | Nonaqueous electrolyte solution 18-2 | (1-2Na) | 1.0 | VC | 1.0 | 65.8 | 115.7 |
| Example 18-3 | Nonaqueous electrolyte solution 18-3 | (1-4) | 1.0 | VC | 1.0 | 80.1 | 107.2 |
| Comparative Example 18-1 | Comparative nonaqueous electrolyte solution 18-1 | - | - | VC | 1.0 | 100.0 | 100.0 |
| Comparative Example 18-2 | Comparative nonaqueous electrolyte solution 18-2 | X | 1.0 | VC | 1.0 | 108.1 | 101.5 |
| Comparative Example 18-3 | Comparative nonaqueous electrolyte solution 18-3 | Y | 1.0 | VC | 1.0 | 141.2 | 101.8 |

### [Table 19]

**Table 19**

| | Nonaqueous Electrolyte Solution | Component (I) or Comparative Compound | | Other Additive | | Initial Resistance (relative value) | Discharge Capacity Retention Rate (relative value) |
|---|---|---|---|---|---|---|---|
| | | Type | Addition Amount [% by mass] | Type | Addition Amount [% by mass] | | |
| Example 19-1 | Nonaqueous electrolyte solution 19-1 | (1-1) | 1.0 | DTD | 1.0 | 87.1 | 106.4 |
| Example 19-2 | Nonaqueous electrolyte solution 19-2 | (1-2Na) | 1.0 | DTD | 1.0 | 75.7 | 109.4 |
| Example 19-3 | Nonaqueous electrolyte solution 19-3 | (1-4) | 1.0 | DTD | 1.0 | 90.4 | 104.3 |
| Comparative Example 19-1 | Comparative nonaqueous electrolyte solution 19-1 | - | - | DTD | 1.0 | 100.0 | 100.0 |
| Comparative Example 19-2 | Comparative nonaqueous electrolyte solution 19-2 | X | 1.0 | DTD | 1.0 | 109.1 | 101.5 |
| Comparative Example 19-3 | Comparative nonaqueous electrolyte solution 19-3 | Y | 1.0 | DTD | 1.0 | 132.1 | 101.9 |

### [Table 20]

**Table 20**

| | Nonaqueous Electrolyte Solution | Component (I) or Comparative Compound | | Other Additive | | Initial Resistance (relative value) | Discharge Capacity Retention Rate (relative value) |
|---|---|---|---|---|---|---|---|
| | | Type | Addition Amount [% by mass] | Type | Addition Amount [% by mass] | | |
| Example 20-1 | Nonaqueous electrolyte solution 20-1 | (1-1) | 1.0 | NaSO₃F | 1.0 | 93.3 | 105.9 |
| Example 20-2 | Nonaqueous electrolyte solution 20-2 | (1-2Na) | 1.0 | NaSO₃F | 1.0 | 79.9 | 109.7 |
| Example 20-3 | Nonaqueous electrolyte solution 20-3 | (1-4) | 1.0 | NaSO₃F | 1.0 | 95.8 | 104.2 |
| Comparative Example 20-1 | Comparative nonaqueous electrolyte solution 20-1 | - | - | NaSO₃F | 1.0 | 100.0 | 100.0 |
| Comparative Example 20-2 | Comparative nonaqueous electrolyte solution 20-2 | X | 1.0 | NaSO₃F | 1.0 | 115.1 | 99.8 |
| Comparative Example 20-3 | Comparative nonaqueous electrolyte solution 20-3 | Y | 1.0 | NaSO₃F | 1.0 | 145.8 | 100.3 |

### [Table 21]

**Table 21**

| | Nonaqueous Electrolyte Solution | Component (I) or Comparative Compound | | Other Additive | | Initial Resistance (relative value) | Discharge Capacity Retention Rate (relative value) |
|---|---|---|---|---|---|---|---|
| | | Type | Addition Amount [% by mass] | Type | Addition Amount [% by mass] | | |
| Example 21-1 | Nonaqueous electrolyte solution 21-1 | (1-1) | 1.0 | TFOP-Na | 1.0 | 81.7 | 107.9 |
| Example 21-2 | Nonaqueous electrolyte solution 21-2 | (1-2Na) | 1.0 | TFOP-Na | 1.0 | 73.4 | 113.4 |
| Example 21-3 | Nonaqueous electrolyte solution 21-3 | (1-4) | 1.0 | TFOP-Na | 1.0 | 84.7 | 107.7 |
| Comparative Example 21-1 | Comparative nonaqueous electrolyte solution 21-1 | - | - | TFOP-Na | 1.0 | 100.0 | 100.0 |
| Comparative Example 21-2 | Comparative nonaqueous electrolyte solution 21-2 | X | 1.0 | TFOP-Na | 1.0 | 108.2 | 101.3 |
| Comparative Example 21-3 | Comparative nonaqueous electrolyte solution 21-3 | Y | 1.0 | TFOP-Na | 1.0 | 131.9 | 102.3 |

### [Table 22]

**Table 22**

| | Nonaqueous Electrolyte Solution | Component (I) or Comparative Compound | | Other Additive | | Initial Resistance (relative value) | Discharge Capacity Retention Rate (relative value) |
|---|---|---|---|---|---|---|---|
| | | Type | Addition Amount [% by mass] | Type | Addition Amount [% by mass] | | |
| Example 22-1 | Nonaqueous electrolyte solution 22-1 | (1-1) | 1.0 | DFP-Na | 1.0 | 83.7 | 109.9 |
| Example 22-2 | Nonaqueous electrolyte solution 22-2 | (1-2Na) | 1.0 | DFP-Na | 1.0 | 74.6 | 114.5 |
| Example 22-3 | Nonaqueous electrolyte solution 22-3 | (1-4) | 1.0 | DFP-Na | 1.0 | 87.3 | 107.3 |
| Comparative Example 22-1 | Comparative nonaqueous electrolyte solution 22-1 | - | - | DFP-Na | 1.0 | 100.0 | 100.0 |
| Comparative Example 22-2 | Comparative nonaqueous electrolyte solution 22-2 | X | 1.0 | DFP-Na | 1.0 | 114.1 | 100.6 |
| Comparative Example 22-3 | Comparative nonaqueous electrolyte solution 22-3 | Y | 1.0 | DFP-Na | 1.0 | 139.7 | 102.2 |

### [Table 23]

**Table 23**

| | Nonaqueous Electrolyte Solution | Component (I) or Comparative Compound | | Other Additive | | Initial Resistance (relative value) | Discharge Capacity Retention Rate (relative value) |
|---|---|---|---|---|---|---|---|
| | | Type | Addition Amount [% by mass] | Type | Addition Amount [% by mass] | | |
| Example 23-1 | Nonaqueous electrolyte solution 23-1 | (1-1) | 1.0 | DFOB-Na | 1.0 | 87.1 | 107.7 |
| Example 23-2 | Nonaqueous electrolyte solution 23-2 | (1-2Na) | 1.0 | DFOB-Na | 1.0 | 79.4 | 112.5 |
| Example 23-3 | Nonaqueous electrolyte solution 23-3 | (1-4) | 1.0 | DFOB-Na | 1.0 | 90.7 | 106.2 |
| Comparative Example 23-1 | Comparative nonaqueous electrolyte solution 23-1 | - | - | DFOB-Na | 1.0 | 100.0 | 100.0 |
| Comparative Example 23-2 | Comparative nonaqueous electrolyte solution 23-2 | X | 1.0 | DFOB-Na | 1.0 | 117.1 | 100.8 |
| Comparative Example 23-3 | Comparative nonaqueous electrolyte solution 23-3 | Y | 1.0 | DFOB-Na | 1.0 | 128.5 | 101.4 |

As it is clear from Tables 1 to 23, it can be seen that the nonaqueous electrolyte solution batteries using the nonaqueous electrolyte solutions containing the component (I) of the present disclosure can decrease the initial resistance. In addition, it can be seen that the nonaqueous electrolyte solutions containing the component (I) of the present disclosure have high-temperature cycle characteristics equal to or higher than those of the nonaqueous electrolyte solutions of the Comparative Examples which do not contain the component (I).

In addition, it was found that, when the nonaqueous electrolyte solutions using the compounds represented by the general formula (1) described above as the component (I) were compared, the nonaqueous electrolyte solution using the Compound (1-2), which had two -SO₂F groups and did not contain any -SO₂- moiety in the groups other than the -SO₂F groups, had the highest reducing effect of the initial resistance and the highest high-temperature cycle characteristic of the battery, and favorable results were obtained in the order of the Compound (1-1), which had one - SO₂F group and did not contain any -SO₂- moiety in the groups other than the -SO₂F group, the Compound (1-4), which had two -SO₂F groups and contained a -SO₂- moiety in a group other than the -SO₂F groups, and the Compound (1-5), which had one -SO₂F group and contained -SO₂- moieties in groups other than the -SO₂F group.

It was found that, when the nonaqueous electrolyte solutions using the compounds represented by the general formula (2) described above as the component (I) were compared, the nonaqueous electrolyte solution using the Compound (2-1), in which a group other than the -SO₂F groups was a lithium ion, had superior reducing effect of the initial resistance and superior high-temperature cycle characteristic of the battery to those of the nonaqueous electrolyte solution using the Compound (2-2), in which a group other than the -SO₂F groups was an ethyl group, while both of the Compounds (2-1) and (2-2) were compounds having two -SO₂F groups.

### INDUSTRIAL APPLICABILITY

According to the present disclosure, a nonaqueous electrolyte solution which can reduce an initial resistance value of a battery and a nonaqueous electrolyte solution battery can be provided. Moreover, compounds which can be suitably used in the nonaqueous electrolyte solution can be provided.

Although the present disclosure has been explained in detail and referring to specific embodiments, it will be apparent to a person skilled in the art that various changes and modifications can be made without departing from the spirit and the scope of the present disclosure.

The present application is based on a Japanese Patent Application (No. 2023-016465) filed on February 6, 2023, the contents of which are incorporated herein by reference.

## Claims

1. A nonaqueous electrolyte solution, comprising at least one selected from the group consisting of a compound represented by the following general formula (1) and a compound represented by the following general formula (2): 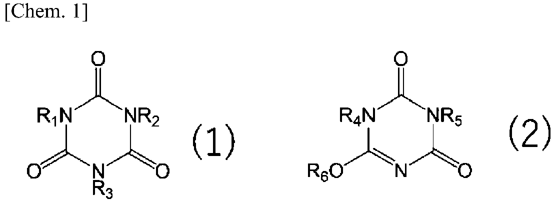
[wherein in the general formula (1), R₁ to R₃ represent groups which may be the same or different and at least one of R₁ to R₃ represents a -SO₂F group, and
in the general formula (2), R₄ to R₆ represent groups which may be the same or different and at least one of R₄ to R₆ represents a -SO₂F group.]

2. The nonaqueous electrolyte solution according to claim 1, wherein one or two of R₁ to R₃ in the general formula (1) represent a -SO₂F group.

3. The nonaqueous electrolyte solution according to claim 1, wherein one or two of R₁ to R₃ in the general formula (1) represent a -SO₂F group, and when a plurality of R₁ to R₃ are other than a -SO₂F group, the plurality of R₁ to R₃ each independently represent a group selected from the group consisting of a hydrogen atom, an alkali metal cation, an alkyl group, an alkenyl group, and an alkynyl group.

4. The nonaqueous electrolyte solution according to claim 1, wherein R₁ to R₃ in the general formula (1) all represent a -SO₂F group.

5. The nonaqueous electrolyte solution according to claim 1, wherein one or two of R₄ to R₆ in the general formula (2) represent a -SO₂F group.

6. The nonaqueous electrolyte solution according to claim 1, wherein R₆ in the general formula (2) represents a -SO₂F group.

7. A nonaqueous electrolyte solution battery, at least comprising:
a positive electrode;
a negative electrode;
a separator; and
the nonaqueous electrolyte solution according to any one of claims 1 to 6.

8. A compound represented by the following general formula (11): 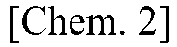 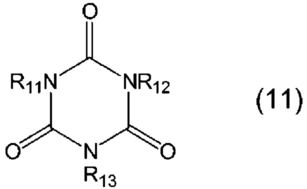 [wherein in the general formula (11), one or two of R₁₁ to R₁₃ represent a -SO₂F group, and when a plurality of R₁₁ to R₁₃ are other than a -SO₂F group, the plurality of R₁₁ to R₁₃ each independently represent a group selected from the group consisting of a hydrogen atom, an alkali metal cation, an alkyl group, an alkenyl group, and an alkynyl group.]

9. A compound represented by the following general formula (2): 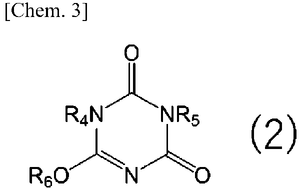 [wherein in the general formula (2), R₄ to R₆ represent groups which may be the same or different, and at least one of R₄ to R₆ represents a -SO₂F group.]
